# EUROPEAN PATENT APPLICATION

(11) **EP 4 745 227 A1**
(43) Date of publication of application: **20.05.2026**
(21) Application number: 24843041.5
(22) Date of filing: 10.07.2024
(51) Int. Cl.: C12N 5/00, C12M 1/00, C12M 3/00, C12N 5/071, C12N 11/08

(54) **GRAFT METHOD, CULTURE CARRIER SUBSTRATE, PACKAGE, CULTURE CARRIER SUBSTRATE EQUIPPED WITH CELL SHEET, METHOD FOR PRODUCING CULTURE CARRIER SUBSTRATE EQUIPPED WITH CELL SHEET, CRYOPRESERVATION METHOD, AND FROZEN PRODUCT OF CULTURE CARRIER SUBSTRATE EQUIPPED WITH CELL SHEET**

(30) Priority: 14.07.2023 JP 2023115623; 27.12.2023 JP 2023220506
(71) Applicant: Central Glass Company, Limited, Ube-shi, Yamaguchi 755-0001 (JP); Yamaguchi University, Yamaguchi 753-8511 (JP)
(72) Inventor: KAWAI Wataru, Tokyo 101-0054 (JP); YOSHIDA Keita, Tokyo 101-0054 (JP); NAKANISHI Yuki, Tokyo 101-0054 (JP); HAMANO Kimikazu, Ube-shi, Yamaguchi 755-8505 (JP); UENO Koji, Ube-shi, Yamaguchi 755-8505 (JP)
(74) Representative: Mewburn Ellis LLP
(86) International application number: PCT/JP2024/024961
(87) International publication number: WO 2025/018249

(57) **Abstract**

A transplantation method according to the present invention includes a culturing step of culturing a cell sheet on a culturing surface of a culturing carrier substrate, and a transplantation step of, after attaching the cell sheet formed over the culturing surface of the culturing carrier substrate to a transplantation site, peeling off the culturing carrier substrate from the cell sheet.

## Description

### TECHNICAL FIELD

The present invention relates to a transplantation method, a culturing carrier substrate, a package, a culturing carrier substrate with a cell sheet, a method of manufacturing a culturing carrier substrate with a cell sheet, a cryopreservation method, and a frozen product of a culturing carrier substrate with a cell sheet.

### BACKGROUND ART

Various developments have been made in "culture" and "transportation" of a cell sheet, which constitute technical elements of a transplantation technique. For example, the technique disclosed in Patent Document 1 is known as this type of technique.

In the background art of Patent Document 1, it is described that in order to transplant a cell sheet to a target affected part, a series of operations are required, such as peeling off the cell sheet cultured on a temperature-responsive material by a change in temperature, covering the obtained cell sheet with a sheet prepared separately to attach the cell sheet to the sheet, extracting the cell sheet from a vessel in this state, transporting the cell sheet to the affected part, and transplanting (attaching) the cell sheet to the affected part.

On the other hand, in claim 1 and paragraph 0007 of Patent Document 1, as a method of transporting the cell sheet cultured in the cell culture vessel having a temperature-responsive polymer layer, a technique is described in which the bottom of the cell culture vessel is peeled off while the cell sheet is attached thereto, and the cell sheet attached to the bottom is transported together with the peeled bottom.

### RELATED DOCUMENT

### PATENT DOCUMENT

Patent Document 1: Japanese Unexamined Patent Publication No. 2016-013111

### SUMMARY OF THE INVENTION

### TECHNICAL PROBLEM

However, as a result of the studies by the present inventor, it has been found that in the transplantation method using the technique of Patent Document 1, it is necessary to release the adhesion state between the cell sheet and the substrate by the low-temperature treatment in a case of peeling off the cell sheet from the temperature-responsive polymer layer, or the adhesiveness between the substrate and the cell sheet is reduced by the low-temperature treatment, and thus it is difficult to cryopreserve the cell sheet cultured in the cell culture vessel having a temperature-responsive polymer layer in a state of being adhered to the cell culture vessel having a temperature-responsive polymer layer.

### SOLUTION TO PROBLEM

As a result of intensive studies, the present inventor has conceived a new concept of integrating "culture" and "transportation" of the cell sheet, which have been studied individually, and has completed the present invention by concretizing the transplantation method, the culturing carrier substrate, and the like.

According to one aspect of the present invention, the following transplantation method, culturing carrier substrate, package, culturing carrier substrate with a cell sheet, method of manufacturing a culturing carrier substrate with a cell sheet, cryopreservation method, and frozen product of a culturing carrier substrate with a cell sheet are provided.
1. A transplantation method including:
   a culturing step of culturing a cell sheet on a culturing surface of a culturing carrier substrate, and
   a transplantation step of, after attaching the cell sheet formed over the culturing surface of the culturing carrier substrate to a transplantation site, peeling off the culturing carrier substrate from the cell sheet.
2. The transplantation method according to 1.,
   in which a substrate thickness of the culturing carrier substrate is equal to or more than 5 µm and equal to or less than 250 µm,
   the culturing carrier substrate is a polyether ether ketone film or a polyethylene terephthalate film, and
   the culturing surface of the culturing carrier substrate is subjected to a hydrophilization treatment.
3. The transplantation method according to 1. or 2.,
   in which a substrate thickness of the culturing carrier substrate is more than 10 µm, and
   in the culturing step, the culturing carrier substrate functions as a self-supporting film without being fixed to an inside of a culture vessel.
4. The transplantation method according to any one of 1. to 3.,
   in which in the transplantation step, a time from the attachment to the peeling off is equal to or less than 10 minutes.
5. The transplantation method according to any one of 1. to 4.,
   in which an area of the cell sheet obtained by the culturing step is equal to or more than 0.3 cm².
6. A culturing carrier substrate used for transporting a cell sheet cultured over a culturing surface,
   in which the culturing carrier substrate has a substrate thickness equal to or more than 5 µm and equal to or less than 250 µm.
7. The culturing carrier substrate according to 6.,
   in which the substrate thickness of the culturing carrier substrate is more than 10 µm, and
   the culturing carrier substrate functions as a self-supporting film without being fixed to an inside of a culture vessel during culturing of the cell sheet.
8. The culturing carrier substrate according to 6. or 7.,
   in which the culturing surface is subjected to a hydrophilization treatment.
9. The culturing carrier substrate according to 8.,
   in which the hydrophilization treatment is a plasma treatment.
10. The culturing carrier substrate according to any one of 6. to 9.,
   in which the culturing carrier substrate is a polyether ether ketone film or a polyethylene terephthalate film.
11. The culturing carrier substrate according to any one of 6. to 10.,
   in which the culturing surface does not contain a temperature-responsive polymer.
12. A package in which the culturing carrier substrate according to any one of 6. to 11. is packaged with a packaging material.
13. A culturing carrier substrate with a cell sheet, comprising:
   a laminate of the culturing carrier substrate according to any one of 6. to 11. and a cell sheet,
   in which the cell sheet is in a state of covering at least 50% of a surface of the culturing carrier substrate on a culturing surface side.
14. The culturing carrier substrate with a cell sheet according to 13.,
   in which an area of the cell sheet is equal to or more than 0.3 cm².
15. A method of manufacturing a culturing carrier substrate with a cell sheet, the method comprising:
   a culturing step of introducing a plurality of cells, a culture medium, and the culturing carrier substrate according to any one of 6. to 11. into an inside of a culture vessel to culture a cell sheet over a culturing surface of the culturing carrier substrate; and
   an extraction step of extracting the culturing carrier substrate with the cell sheet from the culture vessel.
16. The method of manufacturing a culturing carrier substrate with a cell sheet according to 15.,
   in which in the culturing step, the cell sheet is cultured without fixing the culturing carrier substrate to the inside of the culture vessel.
17. The method of manufacturing a culturing carrier substrate with a cell sheet according to 15. or 16.,
   in which in the extraction step, an operation of separating the culturing carrier substrate from the culture vessel by a physical operation is not required.
18. A cryopreservation method comprising a cooling step of cooling, in a cryopreservation solution, a culturing carrier substrate with a cell sheet, said culturing carrier substrate with a cell sheet including a culturing carrier substrate and a cell sheet formed on a surface of the culturing carrier substrate.
19. The cryopreservation method according to 18.,
   in which in the cooling step, the culturing carrier substrate with a cell sheet is cooled in the cryopreservation solution using a non-flow-through-type cooling device.
20. The cryopreservation method according to 18. or 19.,
   in which in the cooling step, a cooling rate at 0°C to -5°C is equal to or more than 0.1°C/min and equal to or less than 15°C/min.
21. The cryopreservation method according to any one of 18. to 20., further comprising:
   a cryopreservation step of storing the culturing carrier substrate with a cell sheet at -196°C to -60°C after the cooling step.
22. A frozen product of a culturing carrier substrate with a cell sheet, the frozen product comprising:
   a culturing carrier substrate; and
   a cell sheet formed on a surface of the culturing carrier substrate,
   in which the culturing carrier substrate and the cell sheet are in a cryopreserved state.
23. The frozen product of a culturing carrier substrate with a cell sheet according to 22.,
   in which the frozen product is in a cryopreserved state at -196°C to -60°C.
24. The frozen product of a culturing carrier substrate with a cell sheet according to 22.,
   in which the frozen product is in a cryopreserved state at -196°C to -135°C.
25. The frozen product of a culturing carrier substrate with a cell sheet according to 22.,
   in which the frozen product is in a cryopreserved state at -134°C to -60°C.
26. A package in which the frozen product according to any one of 22 to 25 is packaged with a packaging material.

### ADVANTAGEOUS EFFECTS OF INVENTION

According to the present invention, a transplantation method capable of performing both culture and transportation of a cell sheet, a culturing carrier substrate, a package, a culturing carrier substrate with a cell sheet, a method of manufacturing a culturing carrier substrate with a cell sheet, a cryopreservation method, and a frozen product of a culturing carrier substrate with a cell sheet are provided.

### BRIEF DESCRIPTION OF THE DRAWINGS

FIGs. 1(A) to 1(E) are schematic step cross-sectional views showing an example of steps of a transplantation method according to the present embodiment.
FIG. 2 i a schematic cross-sectional view showing an example of a configuration of a culturing carrier substrate with a cell sheet according to the present embodiment.
FIGs. 3 (A) to 3(C) are schematic step cross-sectional views showing a modification example of a freezing method according to the present embodiment.

### DESCRIPTION OF EMBODIMENTS

Hereinafter, a description will be made for an embodiment of the present invention using the drawings. It is noted that in all the drawings, the same constitutional elements are denoted as the same reference numerals and explanations thereof will not be repeated, as appropriate. In addition, the figures are schematic views and do not correspond to the actual dimensional ratios.

### <Transplantation Method>

The transplantation method according to the present embodiment includes a culturing step of culturing a cell sheet on a culturing surface of a culturing carrier substrate, and a transplantation step of attaching the cell sheet formed on the culturing surface of the culturing carrier substrate to a transplantation site and then peeling off the culturing carrier substrate from the cell sheet.

Examples of the transplantation site include at least a part of a body of a transplant recipient such as mammals, birds, amphibians, fish, insects, plants, and microorganisms. Specific examples of the mammals and the birds include humans, monkeys, chimpanzees, cows, horses, pigs, sheep, goats, rabbits, dogs, cats, guinea pigs, hamsters, mice, rats, and chickens. However, the transplantation site may be excluded from the inside and outside of the human body. Examples of the transplantation site other than the transplant recipient include other cell sheets, medical instruments such as medical devices, and a tissue or an organ alone that has been separated from the transplant recipient. Examples of the tissue or the organ alone include skin, oral tissue, esophagus, trachea, bronchus, lung, lung lobe, stomach, duodenum, pancreas, spleen, small intestine, large intestine, muscle tissue, and bone (excluding those that are collected from the transplant recipient and returned to the same person for treatment).

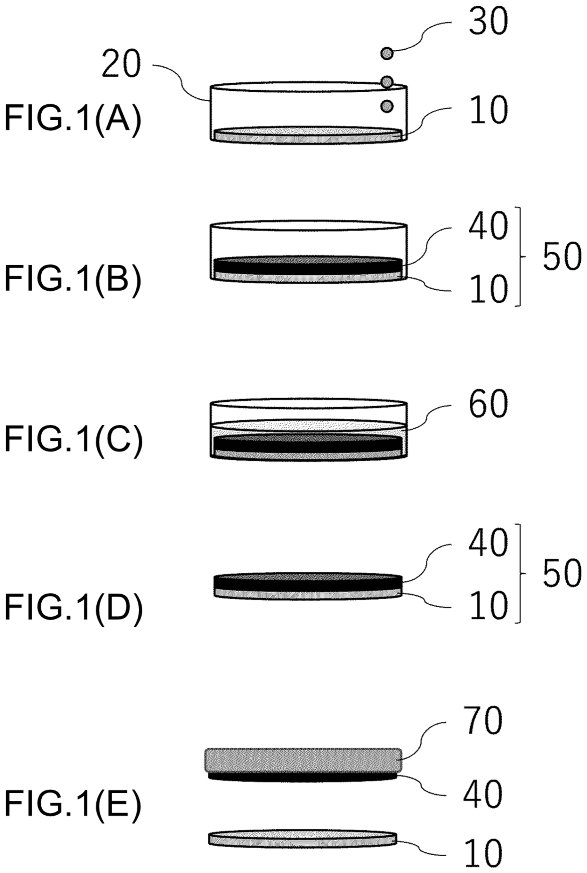

The transplantation method according to the present embodiment will be specifically described with reference to FIGs. 1(A) to 1(E).

FIGs. 1(A) to 1(E) are schematic step cross-sectional views showing an example of steps of the transplantation method. In FIG. 1(A) indicates a cell seeding process, FIG. 1(B) indicates a cell culturing process, FIG. 1(C) indicates a cryopreservation and thawing process, FIG. 1(D) indicates an extraction process, and FIG. 1(E) indicates an attachment process. The cryopreservation and thawing of FIG. 1(C) is an optional process. Here, a transplantation method in which FIG. 1(C) is not performed will be described, and details of FIG. 1(C) will be described in <cryopreservation method> below.

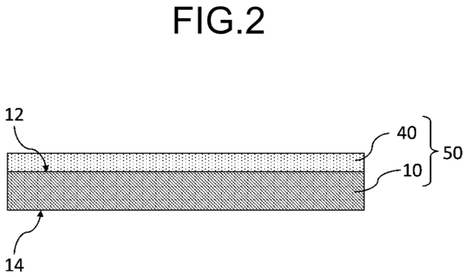

The culturing step may include a cell seeding step shown in FIG. 1(A) and a cell culturing step shown in FIG. 1(B). FIG. 2 is a schematic cross-sectional view of an example of a culturing carrier substrate with a cell sheet 50 obtained in the culturing step.

In the cell seeding step of FIG. 1(A), a culture liquid 30 containing cells is brought into contact with a culturing carrier substrate 10 installed in a culture vessel 20. The cells may be suspended in the culture liquid 30 in advance, or a cell suspension containing the cells may be added to the culture liquid 30.

In the culturing step, a part or the entire part of the culturing carrier substrate 10 may be immersed in the culture liquid 30. Specifically, in the cell seeding step, the culture liquid 30 may be dropped onto a part of the surface of the culturing carrier substrate 10, or the culturing carrier substrate 10 may be immersed in the culture liquid 30 such that at least a part of each of the culturing surface 12 and the side surface of the culturing carrier substrate 10 is brought into contact with the culture liquid 30. The latter immersion method can stably maintain a culture environment in the subsequent cell culturing step and/or can achieve an increase in the area of the culturing surface 12 of the culturing carrier substrate 10, as compared with the former dropping method.

In the cell culturing step of FIG. 1(B), the culture is performed under appropriate culture conditions to form a cell sheet 40 on the culturing surface 12 of the culturing carrier substrate 10. The culture may be performed with a lid (not shown) during the culture.

In the cell culturing step, the cell sheet 40 can be cultured without fixing the culturing carrier substrate 10 to the inside of the culture vessel 20. In this case, the culturing carrier substrate 10 functions as a self-supporting film during the cell culturing. Since the culturing carrier substrate 10 is not fixed, the culturing carrier substrate with a cell sheet 50 can be easily extracted from the culture vessel 20 in the extraction step. In addition, the breakage of the cell sheet 40 during the extraction can also be suppressed. Here, the fixation means a state in which the culturing carrier substrate is adhered to such an extent that a peeling operation by a physical operation is required during the extraction. A fixing mechanism for pressing and fixing the culturing carrier substrate with a load other than the self-weight so as not to move is not included in the "fixation" means referred to here.

In the present specification, the physical operation includes, for example, a method of peeling off while gripping the film, a method of peeling off by scratching with a knife or the like, and the like.

In addition, as one method of immersing the culturing carrier substrate 10 in the culture liquid 30 without fixing the culturing carrier substrate 10, one or two or more of a method of increasing a specific gravity of the culturing carrier substrate 10 to be higher than that of the culture liquid 30, a method of fixing a part of the culturing surface 12 of the culturing carrier substrate 10 by a weight, a method of fixing the position of the culturing carrier substrate 10 by an instrument, or the like may be adopted. That is, in the cell culturing step, the culture can be performed in a state in which the culturing carrier substrate 10 is immersed in the culture liquid 30 by weight fixing and/or position fixing.

The transplantation step may include an extraction step of the culturing carrier substrate with a cell sheet 50 shown in FIG. 1(D) and an attachment step of the cell sheet 40 shown in FIG. 1(E).

In the extraction step of FIG. 1(D), the culturing carrier substrate with a cell sheet 50 is extracted from the culture vessel 20 and is transported to a desired location such as the transplantation site.

In the extraction step according to the present embodiment, the extraction method is not particularly limited, and examples thereof include a method of extracting the culturing carrier substrate 10 constituting the culturing carrier substrate with a cell sheet 50 by pinching, a method of bringing the culturing carrier substrate 10 into contact with a suction nozzle and lifting the culturing carrier substrate 10 while suctioning, and a method of lifting the culturing carrier substrate 10 by threading.

In the attachment step of FIG. 1(E), the cell sheet 40 of the culturing carrier substrate with a cell sheet 50 is attached to a transplantation site 70, and then the culturing carrier substrate 10 is peeled off from the cell sheet 40.

In the present embodiment, in the attachment step, the time from the attachment to the peeling off can be shortened. By shortening the time, the culturing carrier substrate is not exposed to the foreign substance for a long time, so that the risk of an immunological rejection reaction to the substrate film is reduced, the possibility that the cell dies is reduced, and the operation is simplified. The present invention can also be used for surgery in which the chest is opened and the cell sheet is attached to the inside of the body.

The time from the attachment to the peeling off is preferably equal to or less than 10 minutes, more preferably equal to or less than 5 minutes, still more preferably equal to or less than 3 minutes, and even more preferably equal to or less than 1 minute.

Although the detailed mechanism is not clear, it is presumed that since the adhesion between the culturing surface 12 of the culturing carrier substrate 10 and the surface of the cell sheet 40 is of an appropriate strength, the culturing carrier substrate 10 can be peeled off from the cell sheet 40 even before the cell sheet 40 is biologically bonded to the transplantation site 70.

In addition, in the present embodiment, after the peeling, the cell sheet 40 can be suppressed from remaining on the culturing carrier substrate 10. The remaining ratio of the cell sheet 40 on the culturing carrier substrate 10 after the peeling is preferably equal to or less than 50%, more preferably equal to or less than 30%, and still more preferably equal to or less than 10% in terms of area ratio.

The cell transplantation therapy is to suppress and prevent the onset and relapse of symptoms related to the deficiency and functional disorders, and functional failure of cells, tissues, and organs. Examples of the target disease of the cell transplantation therapy include spinal cord injury, knee joint cartilage injury, ischemic heart disease, age-related macular degeneration, corneal epithelial stem cell deficiency, aplastic anemia, severe lower limb ischemia, intractable skin ulcer, prevention of postoperative complications (for example, dehiscence of various organs, bronchial stump fistula, pancreatic fistula, and biliary fistula), and burn.

In addition, the cell used in the cell transplantation therapy may be a self-derived cell, a same-species non-self-derived cell, or a xenogeneic cell. As the preferred cell, from the viewpoint of clinical application and safety, the cell is a self-derived cell, and from the viewpoint of clinical application and productivity, the cell is a non-self-derived cell.

Hereinafter, each element in each step of the transplantation method will be described.

### (Culture Vessel)

The culture vessel 20 is a container for culturing cells in a culture liquid, and is not particularly limited as long as it is suitable for the type and the use of the cells to be cultured.

Examples of the culture vessel 20 include a dish, a petri dish, a tissue culture dish, a multi-dish, a flask, a tissue culture flask, a microplate, a micro-well plate, a multiplate, a multi-well plate, a chamber slide, a dish, a tube, a tray, a culture bag, and a roller bottle.

In addition, the culture vessel 20 may be a culture vessel (for adherent cells) to which a surface-treated cell adhesiveness is imparted or a culture vessel (for suspension cells) which is not surface-treated. The presence or absence of the surface treatment of the culture vessel 20 does not affect the culturing carrier substrate 10. Hereinafter, unless otherwise specified, any of the above can be used.

The material of the culture vessel 20 is not particularly limited as long as the culture liquid 30 is not transmitted therethrough. Examples of the material include polystyrene, polyethylene, polypropylene, polyvinyl alcohol, polyethylene terephthalate, polyacetal, polyvinyl chloride, an acrylic resin, polycarbonate, polyether ether ketone, polyethersulfone, polytetrafluoroethylene, polyimide, polyamide, cellulose, silicone, nylon 6,6, glass, and a metal such as stainless steel or aluminum.

The area of the culture vessel 20 is not particularly limited, and the culture can be performed without any problem as long as the area is that of a commercially available culture vessel. For example, the area is equal to or more than 0.3 cm² and equal to or less than 1000 cm². The lower limit value thereof is more preferably equal to or more than 0.35 cm², still more preferably equal to or more than 1.0 cm², and particularly preferably equal to or more than 1.9 cm². On the other hand, the upper limit value thereof is more preferably equal to or less than 900 cm², still more preferably equal to or less than 800 cm², and particularly preferably equal to or less than 500 cm².

### (Cell)

The cell is not particularly limited as long as it is a clinically useful cell for treating or preventing symptoms related to the deficiency and functional disorders, and functional failure of cells, tissues, and organs, a culture-able cell used in non-clinical tests, or a cell separated from a living body.

Examples of the cell include a living tissue cell, a mesenchymal stromal cell having a capacity to differentiate into a cell belonging to a mesenchymal tissue, a pluripotent stem cell having a capacity to differentiate into various living tissues, and a stem cell or a precursor cell to be induced to differentiate. The cell may be an adherent cell or a suspension cell.

Specific examples of the living tissue cell include a fibroblast, a myofibroblast, a corneal epithelial cell, a retinal cell, a nerve cell, a muscle cell, a myocardial cell, a myoblast, a bone cell, an osteoblast, a chondrocyte, an adipocyte, a hepatocyte, a pancreatic cell, a renal cell, a gingival cell, a periosteal cell, a skin cell, and an endothelial cell.

Specific examples of the mesenchymal stromal cell include a mesenchymal stromal cell derived from adipose tissue, a mesenchymal stromal cell derived from bone marrow, a mesenchymal stromal cell derived from umbilical cord blood, and a mesenchymal stromal cell derived from an umbilical cord.

Specific examples of the pluripotent stem cell include an induced pluripotent stem cell, an embryonic stem cell, a nuclear transfer embryonic stem cell, an embryonal tumor cell, and an embryonal germ cell.

In addition, these cells may be cultured alone or in combination of two or more kinds thereof. For these cells, a known cell may be appropriately set according to the use of the cell.

The origin of the cell is not particularly limited, and examples thereof include mammals, birds, amphibians, fish, insects, plants, and microorganisms. Specific examples of the mammals and the birds include humans, monkeys, chimpanzees, cows, horses, pigs, sheep, goats, rabbits, dogs, cats, guinea pigs, hamsters, mice, rats, and chickens.

### (Culture Conditions)

The cell culturing is not particularly limited, and may be carried out by using a conventional method employed in technical fields such as medicine, pharmaceuticals, quasi-drugs, cosmetics, food, and veterinary drugs, and in basic technical fields such as regenerative medicine and bioengineering.

The culture conditions are not particularly limited as long as the culture cells can be brought into a desired state. As general culture conditions, for example, a 37°C, 5% CO₂ environment using a prepared basal culture liquid is used.

The period of the cell culturing is not particularly limited as long as the culture cells are brought into a desired state. The period of the cell culturing is, for example, within 28 days, within 21 days, within 14 days, within 7 days, within 5 days, or within 3 days. In a case of long-term culturing, the culture medium may be replaced. The frequency and method of the culture medium replacement are not particularly limited. It is generally preferable to replace the culture medium every 1 to 7 days. It is particularly preferably 1 to 5 days. In this case, the entire culture medium may be replaced, or a part of the culture medium may be left and a new culture medium may be added.

The density of the cells to be cultured is not particularly limited as long as it is suitable for the cells to be cultured, the culture vessel, and the use of the culture cells, and is, for example, equal to or more than 5 × 10² cells/cm² and equal to or less than 1 × 10⁹ cells/cm². The lower limit value of the cell density is more preferably equal to or more than 1 × 10³ cells/cm², still more preferably equal to or more than 5 × 10³ cells/cm², and particularly preferably equal to or more than 5 × 10⁴ cells/cm². On the other hand, the upper limit value of the cell density is more preferably equal to or less than 1 × 10⁸ cells/cm², still more preferably equal to or less than 5 × 10⁷ cells/cm², and particularly preferably equal to or less than 1 × 10⁷ cells/cm².

The density of the cells to be cultured is, for example, equal to or more than 5 × 10² cells/cm² and equal to or less than 1 × 10⁹ cells/cm², equal to or more than 5 × 10² cells/cm² and equal to or less than 1 × 10⁸ cells/cm², equal to or more than 5 × 10² cells/cm² and equal to or less than 5 × 10⁷ cells/cm², equal to or more than 5 × 10² cells/cm² and equal to or less than 1 × 10⁷ cells/cm², equal to or more than 1 × 10³ cells/cm² and equal to or less than 1 × 10⁹ cells/cm², equal to or more than 1 × 10³ cells/cm² and equal to or less than 1 × 10⁸ cells/cm², equal to or more than 1 × 10³ cells/cm² and equal to or less than 5 × 10⁷ cells/cm², equal to or more than 1 × 10³ cells/cm² and equal to or less than 1 × 10⁷ cells/cm², equal to or more than 5 × 10³ cells/cm² and equal to or less than 1 × 10⁹ cells/cm², equal to or more than 5 × 10³ cells/cm² and equal to or less than 1 × 10⁸ cells/cm², equal to or more than 5 × 10³ cells/cm² and equal to or less than 5 × 10⁷ cells/cm², equal to or more than 5 × 10³ cells/cm² and equal to or less than 1 × 10⁷ cells/cm², equal to or more than 5 × 10⁴ cells/cm² and equal to or less than 1 × 10⁹ cells/cm², equal to or more than 5 × 10⁴ cells/cm² and equal to or less than 1 × 10⁸ cells/cm², equal to or more than 5 × 10⁴ cells/cm² and equal to or less than 5 × 10⁷ cells/cm², or equal to or more than 5 × 10⁴ cells/cm² and equal to or less than 1 × 10⁷ cells/cm².

### (Culture Liquid)

The culture liquid 30 is not particularly limited as long as it is suitable for the cells to be cultured. Examples of the culture liquid components include sugars, amino acids, vitamins, inorganic salts, trace metals, and additives.

In addition, these culture liquid components may be compounded alone or in combination of two or more kinds thereof. For these culture liquid components, a known component may be appropriately set according to the cells to be cultured.

Specific examples of the sugars include monosaccharides such as glucose, fructose, mannose, and galactose, disaccharides such as sucrose, sucralose, trehalose, maltose, and lactose, trisaccharides such as glucosyl sucrose, lactulosucrose, and raffinose, tetrasaccharides such as acarbose and maltotetraose, cyclodextrin, and oligosaccharides.

Specific examples of the amino acid include L-glutamic acid, L-glutamine, L-arginine, L-cystine, glycine, L-histidine, L-isoleucine, L-leucine, L-lysine, L-methionine, L-phenylalanine, L-serine, L-threonine, L-tryptophan, L-tyrosine, L-valine, L-alanine, L-asparagine, L-aspartic acid, L-cysteine, and L-hydroxyproline.

Specific examples of the vitamins include L-ascorbic acid sodium, L-ascorbic acid-diphosphoric acid, choline, folic acid, niacin, biotin, pantothenic acid, pyridoxine, riboflavin, thiamine, thymidine, and vitamin B12.

Specific examples of the inorganic salt include sodium chloride, sodium hydroxide, sodium sulfate, sodium phosphate, disodium hydrogen phosphate, sodium carbonate, sodium hydrogen carbonate, potassium chloride, potassium hydroxide, potassium sulfate, potassium phosphate, potassium hydrogen phosphate, potassium carbonate, potassium hydrogen carbonate, calcium chloride, calcium sulfate, calcium nitrate, calcium phosphate, calcium carbonate, magnesium chloride, magnesium sulfate, magnesium nitrate, magnesium phosphate, and magnesium carbonate.

Specific examples of the trace metal include iron sulfate, iron nitrate, copper sulfate, copper nitrate, and zinc sulfate.

Specific examples of the additive include serum such as bovine serum, equine serum, and human serum; growth factors such as FGF2, EGF, HGF, VEGF, and PDGF; proteins such as albumin; antioxidants such as glutathione, ascorbic acid, and ascorbic acid derivatives; antibiotics such as penicillin and streptomycin; pH adjusters such as HEPES; organic acids such as lactic acid and propionic acid; lipids such as cholesterol; fatty acids such as linolenic acid; amines such as ethanolamine and putrescine; reducing agents such as mercaptoethanol and 3-mercapto-1,2-propanediol; thickening agents such as sodium alginate, polyvinylpyrrolidone, carboxymethyl cellulose, and pullulan; and pH indicators such as phenol red.

Examples of the culture liquid 30 containing the above-described culture liquid components include a basal culture liquid such as AIM V medium, HFDM-1 Medium, a balanced buffer solution such as Dulbecco's phosphate buffered saline (D-PBS) or Hank's balanced salt solution (HBSS), Dulbecco's Modified Eagle Medium (DMEM) or Eagle's Minimum Essential Medium (EMEM), α-MEM (Minimum Essential Medium alpha Modification), Iscove's Modified Dulbecco's Medium (IMDM), Glasgow's MEM (GMEM), Ham's F-10 medium, Ham's F-12 medium, Ham's F-12K medium, RPMI medium 1640, M-199 medium, L-15 medium, McCoy's 5A Medium, MCDB105 medium, MCDB107 medium, MCDB131 medium, MCDB153 medium, MCDB201 medium, NCTC 109 medium, NCTC 135 medium, Waymouth's MB752/1 medium, CMRL-1066 medium, Williams' medium E, Brinster's BMOC-3 Medium, and E8 medium.

In addition, these basal culture liquids may be used alone or in combination of two or more kinds thereof. Furthermore, the basal culture liquid may be added, removed, increased, or decreased in the culture liquid components according to the type or the state of the cell. For these basal culture liquids, a known culture liquid may be appropriately set according to the cells to be cultured.

### (Cell Sheet)

The cell sheet 40 is a sheet structure in which cells are physically and functionally linked to each other through adhesion molecules, an extracellular matrix, or the like.

The cell sheet 40 may have a monolayer structure composed of one cell layer or a laminated structure composed of two or more cell layers. The laminated structure is not particularly limited, and examples thereof include a multilayer structure of two layers, three layers, four layers, or five layers.

In a case where the cell sheet 40 has a multilayer structure, the multilayer structure may be obtained in a case of being cultured with the culturing carrier substrate 10, or the cell sheet having a monolayer structure may be laminated to obtain the multilayer structure. In particular, by preparing a plurality of culturing carrier substrates with a cell sheet 50 according to the embodiment of the present invention, laminating another cell sheet on a certain cell sheet, and peeling off the culturing carrier substrate from the other cell sheet, a cell sheet having a multilayer structure can be obtained.

The thickness of the cell sheet 40 is not particularly limited, and is, for example, equal to or more than 0.001 mm and equal to or less than 2.0 mm. The lower limit value of the thickness of the cell sheet 40 is more preferably equal to or more than 0.01 mm, still more preferably equal to or more than 0.03 mm, and particularly preferably equal to or more than 0.05 mm. On the other hand, the upper limit value of the thickness of the cell sheet 40 is more preferably equal to or less than 1.5 mm, still more preferably equal to or less than 1.2 mm, and particularly preferably equal to or less than 1.0 mm. The thickness of the cell sheet 40 is, for example, equal to or more than 0.001 mm and equal to or less than 2.0 mm, equal to or more than 0.001 mm and equal to or less than 1.5 mm, equal to or more than 0.001 mm and equal to or less than 1.2 mm, equal to or more than 0.001 mm and equal to or less than 1.0 mm, equal to or more than 0.01 mm and equal to or less than 2.0 mm, equal to or more than 0.01 mm and equal to or less than 1.5 mm, equal to or more than 0.01 mm and equal to or less than 1.2 mm, equal to or more than 0.01 mm and equal to or less than 1.0 mm, equal to or more than 0.03 mm and equal to or less than 2.0 mm, equal to or more than 0.03 mm and equal to or less than 1.2 mm, equal to or more than 0.03 mm and equal to or less than 1.0 mm, equal to or more than 0.05 mm and equal to or less than 2.0 mm, equal to or more than 0.05 mm and equal to or less than 1.5 mm, equal to or more than 0.05 mm and equal to or less than 1.2 mm, or equal to or more than 0.05 mm and equal to or less than 1.0 mm. By setting the thickness of the cell sheet 40 within the above-described range, it is possible to exhibit a high cell viability and excellent shape retention ability advantageous for cell transplantation in the cell sheet 40.

The area of the cell sheet 40 is not particularly limited, and is, for example, equal to or more than 0.3 cm² and equal to or less than 1000 cm². The lower limit value of the area of the cell sheet 40 is more preferably equal to or more than 0.6 cm², still more preferably equal to or more than 1.6 cm², and particularly preferably equal to or more than 8 cm², and may be equal to or more than 3 cm², equal to or more than 6 cm², or equal to or more than 10 cm². On the other hand, the upper limit value of the area of the cell sheet 40 is more preferably equal to or less than 900 cm², still more preferably equal to or less than 800 cm², and particularly preferably equal to or less than 500 cm², and may be equal to or less than 100 cm², equal to or less than 50 cm², or equal to or less than 20 cm². For example, the area of the cell sheet 40 is equal to or more than 0.6 cm² and equal to or less than 900 cm², equal to or more than 1.6 cm² and equal to or less than 800 cm², equal to or more than 3 cm² and equal to or less than 500 cm², equal to or more than 6 cm² and equal to or less than 100 cm², equal to or more than 8 cm² and equal to or less than 50 cm², or equal to or more than 10 cm² and equal to or less than 20 cm². In the related art, in a case of transplanting the cell sheet alone, the strength of the cell sheet is low, so that the cell sheet is likely to be damaged during transport in a case of a large area. However, in the present disclosure, since the cell sheet 40 is supported by the culturing carrier substrate, the cell sheet can be prevented from breaking during the transplantation, so that the size of the cell sheet 40 can be increased to 8 cm² or more. In addition, the cell sheet can be prepared to be large and used as appropriate for the size of the affected part.

In FIGs. 1(A) to 1(E), the shape of the culturing carrier substrate is shown as a circle, but the shape of the culturing carrier substrate may be any appropriate shape other than a circle, for example, a regular polygon such as a square, a regular pentagon, a regular hexagon, or a regular octagon, or an oval or a rectangle.

### <Culturing Carrier Substrate>

The culturing carrier substrate 10 according to the present embodiment is a substrate that is used for both a cell sheet scaffold for culturing cells to form a cell sheet 40 and a cell sheet support for transporting the cultured cell sheet 40, and that is peeled off from the cell sheet after the cell sheet is attached to a transplantation site.

The area of the culturing carrier substrate 10 is not particularly limited, but is desirably smaller than the culture vessel and the same as or larger than the cell sheet. For example, the area is equal to or more than 0.3 cm² and equal to or less than 1000 cm². The lower limit value of the area of the culturing carrier substrate 10 is more preferably equal to or more than 0.6 cm², still more preferably equal to or more than 1.6 cm², and particularly preferably equal to or more than 8 cm², and may be equal to or more than 3 cm², equal to or more than 6 cm², or equal to or more than 10 cm². On the other hand, the upper limit value of the area of the culturing carrier substrate 10 is more preferably equal to or less than 900 cm², still more preferably equal to or less than 800 cm², and particularly preferably equal to or less than 500 cm², and may be equal to or less than 100 cm², equal to or less than 50 cm², or equal to or less than 20 cm². In a case where the area is larger than the culture vessel, the cell sheet may not be obtained. For example, the area of the culturing carrier substrate 10 is equal to or more than 0.6 cm² and equal to or less than 900 cm², equal to or more than 1.6 cm² and equal to or less than 800 cm², equal to or more than 3 cm² and equal to or less than 500 cm², equal to or more than 6 cm² and equal to or less than 100 cm², equal to or more than 8 cm² and equal to or less than 50 cm², or equal to or more than 10 cm² and equal to or less than 20 cm².

The substrate thickness of the culturing carrier substrate 10 is not particularly limited, but is preferably equal to or more than 5 µm and equal to or less than 250 µm. The lower limit value of the substrate thickness is more preferably equal to or more than 6 µm, still more preferably equal to or more than 8 µm, equal to or more than 10 µm, or equal to or more than 12 µm. On the other hand, the upper limit value of the substrate thickness is more preferably equal to or less than 50 µm, equal to or less than 30 µm, equal to or less than 25 µm, and still more preferably equal to or less than 20 µm. For example, the substrate thickness of the culturing carrier substrate 10 is equal to or more than 6 µm and equal to or less than 50 µm, equal to or more than 8 µm and equal to or less than 30 µm, or equal to or more than 10 µm and equal to or less than 20 µm.

By setting the substrate thickness to be equal to or larger than the lower limit value, the culturing carrier substrate 10 can be prevented from breaking or being deformed during the transport, and the handleability can be improved. By setting the substrate thickness to be equal to or less than the upper limit value, the followability to the affected part during the transplantation can be improved. In particular, in a case where the substrate thickness is set to equal to or less than 20 µm, the followability to the affected part having a higher curvature can be further improved, and the culturing carrier substrate can be applied to, for example, a site where an organ inside the body is sutured by surgery.

In addition, from the viewpoint of functioning as a self-supporting film during the culture, the lower limit of the substrate thickness of the culturing carrier substrate 10 is preferably more than 10 µm, more preferably equal to or more than 11 µm, and still more preferably equal to or more than 12 µm. As a result, by using the culturing carrier substrate 10 having a substrate thickness of more than 10 µm, the cell sheet 40 can be manufactured in a state where the culturing carrier substrate 10 is not fixed or adhered to the inside of the culture vessel 20 in the cell culturing step.

The self-supporting film is preferably a film in which the planar state of the culturing surface 12 of the culturing carrier substrate 10 is maintained even in the culture liquid 30, and it is more preferable that the planar state is maintained even after the cell sheet 40 is formed on the culturing surface 12. Since the planar state of the culturing surface 12 is maintained, the culturing carrier substrate 10 that is not deformed can improve the cell culturing characteristics in the culture liquid 30 without being fixed.

In addition, the self-supporting film may be able to maintain a sheet shape to some extent in a case where one end thereof is pinched and lifted with forceps. In this case, the area of the culturing carrier substrate 10 is preferably equal to or more than 1.6 cm².

The culturing surface 12 of the culturing carrier substrate 10 is preferably subjected to a hydrophilization treatment. As a result, the cell adhesiveness can be improved. Examples of the hydrophilization treatment include a UV ozone treatment and a plasma treatment.

Among these, it is preferable to use the culturing carrier substrate 10 in which the culturing surface 12 is subjected to the plasma treatment.

In the present specification, the hydrophilic surface means a surface in which the upper limit of the water droplet contact angle θ measured by the θ/2 method is equal to or less than 70°. On the other hand, the lower limit of the water droplet contact angle θ of the hydrophilic surface is preferably equal to or more than 3°, but is not limited thereto. The water droplet contact angle θ is measured by placing 2 µl of pure water on the substrate in an environment of 25°C and measuring an angle formed by the water droplet and the substrate with a contact angle meter according to the θ/2 method in accordance with ISO 19403-2:2017.

In addition, the lower limit of the surface roughness Ra of the hydrophilic-treated culturing surface is equal to or more than 0.3 nm, preferably equal to or more than 0.5 nm, and the upper limit thereof is equal to or less than 100 nm, preferably equal to or less than 10 nm, and more preferably equal to or less than 2 nm.

The surface roughness Ra here refers to an arithmetic average roughness in a square region having one side of 100 nm, using surface shape data measured with an atomic force microscope (AFM).

Examples of a material constituting the culture carrier substrate 10 include resin materials such as polyether ether ketone (PEEK), polyethylene terephthalate (PET), polybutylene terephthalate (PBT), polystyrene (PS), polycarbonate (PC), modified polyphenylene ether (mPPE), polyphenylene sulfide (PPS), polysulfone (PSU), polyarylate (PAR), liquid crystal polymer (LCP), polyethylene (PE), and polypropylene (PP).

An example of the culturing carrier substrate 10 may be composed of a film containing at least one of these resin materials as a main component, and is preferably a polyether ether ketone film or a polyethylene terephthalate film, and more preferably a polyether ether ketone film.

The culturing carrier substrate 10 preferably includes one or two or more resin layers consisting of the above-described resin material. The two or more resin layers may contain different types of materials.

The culturing carrier substrate 10 may have a configuration consisting of a resin substrate including a resin layer consisting of the above-described material, or may have a configuration consisting of a laminate substrate including a resin layer consisting of the above-described material and an inorganic layer consisting of another material. However, the resin layer may not include a layer formed by chemical vapor deposition such as parylene or a wet coating layer. In addition, the inorganic layer in the laminate substrate preferably does not include a glass layer, and may include a metal foil. In addition, the culturing carrier substrate 10 preferably does not include a nonwoven fabric and a fiber substrate on the culturing surface 12 side.

In a case where the culturing carrier substrate 10 including PEEK is comprehensively considered from the viewpoint of a low coefficient of thermal expansion, solvent resistance, and heat resistance, the culturing carrier substrate 10 including PEEK is preferable as compared with a case of including PET.

In addition, the culturing carrier substrate 10 is preferably composed of a material having a higher specific gravity than the culture liquid 30.

The culturing carrier substrate 10 is a culturing carrier substrate in which, in a case where the culturing surface 12 of the culturing carrier substrate 10 is measured with a laser microscope, there are three or less holes having a diameter equal to or more than 1 µm and equal to or less than 100 µm and a depth equal to or more than 0.5 µm and equal to or less than 100 µm in a range of 100 µm square. The diameter of the hole is, for example, equal to or more than 1 µm and equal to or less than 100 µm, preferably equal to or more than 2 µm and equal to or less than 50 µm, and more preferably equal to or more than 3 µm and equal to or less than 30 µm. The depth of the hole is, for example, equal to or more than 0.5 µm and equal to or less than 100 µm, preferably equal to or more than 1 µm and equal to or less than 50 µm, and more preferably equal to or more than 2 µm and equal to or less than 20 µm.

A combination of the diameter of the hole and the range of the depth of the hole is, for example, a combination of a diameter of the hole equal to or more than 1 µm and equal to or less than 100 µm and a depth of the hole equal to or more than 0.5 µm and equal to or less than 100 µm, a diameter of the hole equal to or more than 1 µm and equal to or less than 100 µm and a depth of the hole equal to or more than 1 µm and equal to or less than 50 µm, a diameter of the hole equal to or more than 1 µm and equal to or less than 100 µm and a depth of the hole equal to or more than 2 µm and equal to or less than 20 µm, a diameter of the hole equal to or more than 2 µm and equal to or less than 50 µm and a depth of the hole equal to or more than 0.5 µm and equal to or less than 100 µm, a diameter of the hole equal to or more than 2 µm and equal to or less than 50 µm and a depth of the hole equal to or more than 1 µm and equal to or less than 50 µm, a diameter of the hole equal to or more than 2 µm and equal to or less than 50 µm and a depth of the hole equal to or more than 2 µm and equal to or less than 20 µm, a diameter of the hole equal to or more than 3 µm and equal to or less than 30 µm and a depth of the hole equal to or more than 0.5 µm and equal to or less than 100 µm, a diameter of the hole equal to or more than 3 µm and equal to or less than 30 µm and a depth of the hole equal to or more than 1 µm and equal to or less than 50 µm, or a diameter of the hole equal to or more than 3 µm and equal to or less than 30 µm and a depth of the hole equal to or more than 2 µm and equal to or less than 20 µm.

In addition, the upper limit of the void ratio of the culturing carrier substrate 10 is, for example, equal to or less than 15%, preferably equal to or less than 10%, and more preferably equal to or less than 5%. On the other hand, the lower limit of the void ratio of the culturing carrier substrate 10 is not particularly limited, and may be equal to or more than 0%.

By setting the number of holes in the culturing surface 12 to three or less and/or setting the void ratio of the culturing carrier substrate 10 to the equal to or less than upper limit value, the adhesiveness to the cell sheet 40 can be set to an appropriate level.

The presence or absence of the hole may be determined by measuring a surface of the resin layer formed on the culturing surface 12 side of the culturing carrier substrate 10.

The void ratio is calculated from a theoretical density and a measured density. Specifically, the void ratio is calculated by the following calculation expression: void ratio = {1 - (actual density/theoretical density)} × 100.

In addition, the culturing surface 12 of the culturing carrier substrate 10 may be configured not to contain a temperature-responsive polymer. As a result, the adhesiveness between the cell sheet 40 and the culturing carrier substrate 10 can be suppressed from being reduced in a low-temperature environment such as a cryopreservation step.

The temperature-responsive polymer is a material that exhibits cell adhesiveness at a temperature in a case of culturing cells and exhibits cell non-adhesiveness by changing the temperature from there, thereby making it possible to easily peel off the cell sheet. In a case where the temperature range in which the temperature-responsive polymer exhibits cell adhesiveness is 10°C to 45°C, particularly 33°C to 40°C, the cells can be stably cultured, which is preferable. In addition, in a case where the temperature range in which the temperature-responsive polymer exhibits cell non-adhesiveness is 1°C to 36°C, particularly 4°C to 32°C, the damage to the cell sheet during the peeling can be reduced, which is preferable. Specific examples of the material constituting the temperature-responsive polymer include temperature-responsive polymers such as poly-N-isopropylacrylamide (PNIPAAm), poly-N-n-propylacrylamide, poly-N-n-propylmethacrylamide, poly-N-ethoxyethylacrylamide, poly-N-tetrahydrofurfurylacrylamide, poly-N-tetrahydrofurfurylmethacrylamide, and poly-N,N-diethylacrylamide, among which PNIPAAm, poly-N-n-propylmethacrylamide, and poly-N,N-diethylacrylamide are preferable.

The culturing carrier substrate 10 before being used for the culture of the cell sheet can be a package packaged with a packaging material. By sealing the package, the culturing surface can be stored and circulated without being contaminated. In addition, the package may be sterilized. Examples of the sterilization method include electron beam sterilization, gamma ray sterilization, EOG sterilization, and high-pressure steam sterilization.

Examples of the packaging material include aluminum, polyethylene terephthalate, ionomer, polyethylene, polyvinyl chloride, polyvinylidene chloride, polyvinyl alcohol, polypropylene, polyester, polycarbonate, polystyrene, polyacrylonitrile, ethylene vinyl acetate copolymer, ethylene-vinyl alcohol copolymer, ethylene-methacrylic acid copolymer, perfluoroalkoxy fluororesin, nylon, cellophane, and paper materials. These may be used alone or in combination of two or more thereof. In a case of performing sterilization in which gas permeability is required, such as EOG sterilization and high-pressure steam sterilization, a packaging material in which the paper material and the above-described material are combined is preferably used. The packaging material may be multi-packaged, and the packaging may be further overlapped after the sterilization. The outermost part of the package is preferably a film-like packaging material of a resin.

By culturing the cell sheet 40 on the culturing carrier substrate 10, a culturing carrier substrate with a cell sheet 50 including a laminate of the cell sheet 40 and the culturing carrier substrate 10 is obtained.

In the culturing carrier substrate with a cell sheet 50, the cell sheet 40 is in a state of covering, for example, equal to or more than 50% and equal to or less than 100% of a region in which the cells can be deposited on the surface on the culturing surface 12 side. In a case where a method of fixing a part of the culturing surface 12 of the culturing carrier substrate 10 by a weight or a method of fixing the position of the culturing carrier substrate 10 by an instrument is adopted, the region in which the cells can be deposited is a region in which the weight or the instrument does not come into contact and the cells can be deposited. In addition, in a case where the culturing surface 12 of the culturing carrier substrate 10 is subjected to a hydrophilization treatment, the region in which the cells can be deposited is a region subjected to the hydrophilization treatment. The lower limit value of the covering area of the cell sheet 40 is preferably equal to or more than 70% and more preferably equal to or more than 90%. The upper limit value of the covering area of the cell sheet 40 may be equal to or less than 100%, equal to or less than 99%, or equal to or less than 98%. The covering area of the cell sheet 40 is, for example, equal to or more than 50% and equal to or less than 100%, equal to or more than 50% and equal to or less than 99%, equal to or more than 50% and equal to or less than 98%, equal to or more than 70% and equal to or less than 100%, equal to or more than 70% and equal to or less than 99%, equal to or more than 70% and equal to or less than 98%, equal to or more than 90% and equal to or less than 100%, equal to or more than 90% and equal to or less than 99%, or equal to or more than 90% and equal to or less than 98%. The area of the culturing carrier substrate 10 is preferably the same as or larger than the area of the region in which the cells of the cell sheet 40 can be deposited. By setting the covering area to be equal to or larger than the lower limit value, the healing effect can be improved.

In addition, by forming the cell sheet on the culturing carrier substrate, the possibility of breaking or the like in a case of extracting the cell sheet from the culture vessel or the cryopreservation vessel can be reduced, and the cell sheet can be easily extracted. In addition, after the cell sheet is attached to the transplantation site, the cell sheet can be easily peeled off from the culturing carrier substrate. Since the cell sheet is easy to handle, a large-sized cell sheet can be prepared.

### <Method of Manufacturing Culturing Carrier Substrate with Cell Sheet>

An example of the method of manufacturing the culturing carrier substrate with a cell sheet 50 according to the present embodiment may include a culturing step of introducing a plurality of cells, a culture medium (culture liquid 30), and the culturing carrier substrate 10 into the inside of the culture vessel 20 to culture the cell sheet 40 on the culturing surface 12 of the culturing carrier substrate 10, and an extraction step of extracting the culturing carrier substrate with a cell sheet 50 from the culture liquid 30.

The cell sheet 40 according to the present embodiment is peeled off from the culturing carrier substrate with a cell sheet 50, and can be used for treatment, prevention, and the like of a cell transplantation therapy.

The peeling method is not particularly limited, and a usual method used in the technical field of medicine, pharmaceuticals, cosmetics, food, veterinary drugs, and the like, and the basic technology field of regenerative medicine, bioengineering, and the like can be used. Examples of the peeling method include a physical treatment.

### <Cryopreservation Method>

An example of the cryopreservation method according to the present embodiment includes, as shown in FIG. 1(C), a cooling step of cooling the culturing carrier substrate with a cell sheet 50 including the culturing carrier substrate 10 and the cell sheet 40 formed on the surface (culturing surface 12) of the culturing carrier substrate 10 in the inside of the culture vessel 20 in a cryopreservation solution 60. The culture vessel may be frozen with a lid for culture (not shown) or a lid for preventing liquid leakage and microbial contamination (not shown). In FIG. 1(C), the culturing carrier substrate with a cell sheet 50 is cryopreserved in the inside of the culture vessel 20, but the culturing carrier substrate with a cell sheet 50 may be transferred to a cryopreservation vessel different from the culture vessel 20 to be cryopreserved. The cryopreservation vessel is not particularly limited, and for example, the culture vessel 20 can be used.

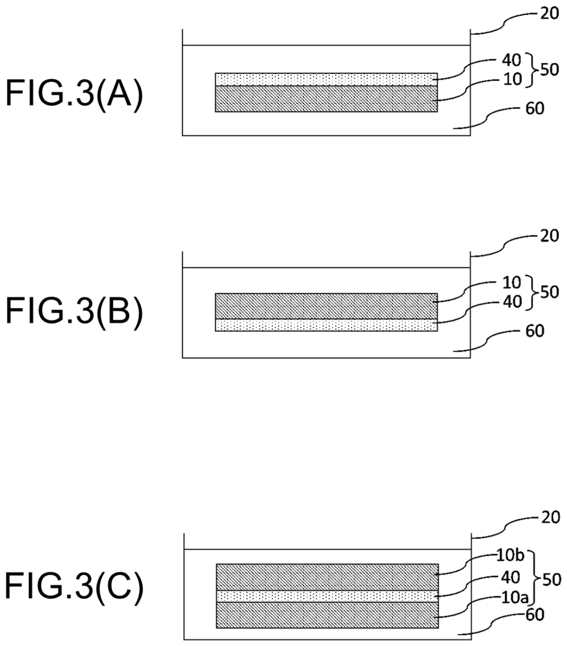

In the cooling step, various aspects can be adopted for the culturing carrier substrate with a cell sheet 50. As shown in FIGs. 3(A) and 3(B), the culturing carrier substrate with a cell sheet 50 may have a structure in which the culturing carrier substrate 10 is formed on one side of the cell sheet 40, or as shown in FIG. 3(C), may have a structure in which both surfaces of the cell sheet 40 are sandwiched between two culturing carrier substrates 10a and 10b. In FIG. 3(A), the culturing carrier substrate 10 is disposed in a state of facing a bottom surface of the culture vessel 20. In FIG. 3(B), the cell sheet 40 is disposed in a state of facing the bottom surface of the culture vessel 20. In FIG. 3(C), the culturing carrier substrates 10a and 10b may be composed of the same material or may be composed of different materials from each other. For example, both of the culturing carrier substrates 10a and 10b may be culturing carrier substrates including PEEK, or one of the culturing carrier substrates 10a and 10b may be a culturing carrier substrate including PEEK and the other may be a culturing carrier substrate not including PEEK.

### (Cryopreservation Solution)

The cryopreservation solution 60 is a solution for reducing damage to the cell due to freezing and storage.

The cryopreservation solution 60 is not particularly limited as long as it is suitable for freezing and storing the cell, but may contain a culture liquid component, a freezing protective agent, and the like contained in the culture liquid.

The sugars, amino acids, vitamins, inorganic salts, trace metals, and additives, which are the culture liquid components, may be those that satisfy the description of the above (culture liquid). In addition, the cryopreservation solution preferably has a coagulation start temperature in a range equal to or higher than -15°C and equal to or lower than -5°C.

The freezing protective agent is a substance used for reducing damage to the cell due to freezing or thawing during cryopreservation. Examples of the freezing protective agent include a cell non-permeable freezing protective agent and a cell permeable freezing protective agent.

Specific examples of the cell non-permeable freezing protective agent include albumin, sucrose, trehalose, dextran, polyethylene glycol, polyvinyl alcohol, polyvinylpyrrolidone, and polylysine.

Specific examples of the cell permeable freezing protective agent include dimethyl sulfoxide (DMSO), glycerol, ethylene glycol, propylene glycol, and propanediol.

In addition, these freezing protective agents may be compounded alone or in combination of two or more kinds thereof. For these freezing protective agents, a known freezing protective agent may be appropriately set according to the type of the cell, the composition of the cryopreservation solution, and the like.

Examples of commercially available DMSO-free cryopreservation solutions include Stem Cell Banker (registered trademark) DMSO-free GMP grade (Nippon Zenyaku Kogyo Co., Ltd.), Bambanker (registered trademark) DMSO-free (GC LYMPHOTEC Inc.), CryoScarless (registered trademark) DMSO-free (Bioverde Co., Ltd.), Stem Cell Keep (Bioverde Co., Ltd.), CryoNovo (registered trademark) X12 (Akron Bioproducts LLC), CryoNovo (registered trademark) P24 (Akron Bioproducts, LLC), DMSO-free cell cryopreservation solution for human ES/iPS cell freezing and storage (ReproCell Inc.), Cell Reservoir One (Nacalai Tesque, Inc.), ThelioKeep (registered trademark: Bioverde Co., Ltd.), Cellvation (registered trademark: Protide Pharmaceuticals), ReproCryo RM (ReproCell Inc.), and SOFORO Cryo (SARAYA Co., Ltd.). In addition, examples of the commercially available cryopreservation solution including DMSO include Stem Cell Banker (registered trademark) GMP grade (Nippon Zenyaku Kogyo Co., Ltd.), Stem Cell Banker (registered trademark) EX GMP grade (Nippon Zenyaku Kogyo Co., Ltd.), Bambanker (registered trademark) hRM (GC LYMPHOTEC Inc.), Bambanker (registered trademark) (GC LYMPHOTEC Inc.), iStock (GC LYMPHOTEC Inc.), CryoStor CS5 (Charles River Laboratories Cell Solutions, Inc.), and CryoStor CS10 (Charles River Laboratories Cell Solutions,

Inc.) .

### (Cooling Step)

In the cooling step, it is preferable that the culturing carrier substrate with a cell sheet 50 is cooled in the cryopreservation solution using a non-flow-through-type cooling device. Since the cell sheet 40 can be cooled at a uniform temperature by the non-flow-through-type cooling device on an appropriate culturing carrier substrate 10, the damage to the cell is small, and the decrease in the cell viability can be suppressed.

In the cooling step, the cooling rate at 0°C to -5°C is, for example, equal to or more than 0.1°C/min and equal to or less than 15°C/min, preferably equal to or more than 0.25°C/min and equal to or less than 12.5°C/min, and more preferably equal to or more than 0.5°C/min and equal to or less than 10°C/min.

By setting the cooling rate to be equal to or larger than the lower limit value, the contact time between the unnecessary liquid freezing protective material and the cell can be reduced. By setting the cooling rate to be equal to or less than the upper limit value, the formation of intracellular ice crystals can be suppressed.

In the cooling step, the temperature of the freezing treatment is not particularly limited as long as the culture cells and the cryopreservation solution can be frozen. The temperature of the freezing treatment is, for example, equal to or higher than -196°C and equal to or lower than -25°C. The lower limit value thereof is more preferably equal to or higher than -180°C, still more preferably equal to or higher than -160°C, and particularly preferably equal to or higher than -150°C. On the other hand, the upper limit value thereof is more preferably equal to or lower than -25°C, still more preferably equal to or lower than -30°C, and particularly preferably equal to or lower than -35°C. The temperature of the freezing treatment is, for example, equal to or higher than -196°C and equal to or lower than -25°C, equal to or higher than -196°C and equal to or lower than -30°C, equal to or higher than -196°C and equal to or lower than -35°C, equal to or higher than -180°C and equal to or lower than -25°C, equal to or higher than -180°C and equal to or lower than -30°C, equal to or higher than -180°C and equal to or lower than -35°C, equal to or higher than -160°C and equal to or lower than -25°C, equal to or higher than -160°C and equal to or lower than -30°C, equal to or higher than -160°C and equal to or lower than -35°C, equal to or higher than -150°C and equal to or lower than -25°C, equal to or higher than -150°C and equal to or lower than -30°C, or equal to or higher than -150°C and equal to or lower than -35°C.

The cooling device used for the freezing treatment is not particularly limited, and examples thereof include a rapid freezing device and an ultra-low temperature refrigerator. As the cooling device, a freezing device that is non-contact with a heat transfer unit and that blows cold air to the culture vessel not in one direction but in multiple directions, preferably in all directions, rather than freezing the culture vessel and the cell by bringing the heat transfer unit into contact with the culture vessel, is preferable from the viewpoint of freezing the cell at a uniform temperature and increasing the survival rate of the cell after thawing. Specific examples of the freezing device that blows the cold air to the culture vessel to freeze the culture vessel include a cooling device that cools a cooling target by circulating the cold air by a cooling fan, for example, a non-flow type cooling device comprising a cooling fan, which is disclosed in Japanese Unexamined Patent Publication No. 2005-127666. The above-described "non-flow type" means a method in which most of the through-flowing air from the cooling target does not pass through (flow through) the cooler.

### (Freezing and Storing Step)

It is preferable that the cryopreservation method includes a cryopreservation step of storing the culturing carrier substrate with a cell sheet 50 at -196°C to -60°C, preferably - 180°C to -65°C, and more preferably -150°C to -80°C after the cooling step. As a result, the cell sheet can be stably maintained for a long period of time.

The temperature of the cooling step may be adopted as a set temperature of the cooling device.

The cryopreservation is not particularly limited as long as the cell can be stably cryopreserved.

Examples of the cryopreservation method include contact with a liquid phase or a gas phase of a coolant, and use of an ultra-low temperature freezer. From the viewpoint of temperature, the preferred cryopreservation method is contact with a liquid phase or a gas phase of a coolant.

Examples of the coolant include liquid nitrogen, liquid ethane, liquid propane, liquid helium, and dry ice.

The temperature of the cryopreservation is not particularly limited as long as the cell can be stably cryopreserved. The temperature of the cryopreservation may be in any of ranges equal to or higher than -196°C and equal to or lower than -60°C, equal to or higher than -196°C and equal to or lower than -134°C, or equal to or higher than -134°C and equal to or lower than -60°C.

The temperature of the cryopreservation may be adopted as a surface temperature of the cell sheet 40 to be frozen. The surface temperature can be measured using, for example, a K-type thermocouple.

The frozen product of the culturing carrier substrate with a cell sheet 50 includes the culturing carrier substrate 10 and the cell sheet 40 formed on the surface (culturing surface 12) of the culturing carrier substrate 10. In the culturing carrier substrate with a cell sheet 50, the culturing carrier substrate 10 and the cell sheet 40 are in a cryopreserved state.

In the frozen product, the culturing carrier substrate 10 and the cell sheet 40 are in a cryopreserved state, for example, at the above-described freezing and storage temperature, specifically, preferably equal to or lower than -60°C and more preferably equal to or lower than -135°C. In addition, the frozen product may be in a cryopreserved state in a range equal to or higher than -134°C and equal to or lower than -60°C. The frozen product is in a cryopreserved state at equal to or higher than -196°C.

The frozen product of the culturing carrier substrate with a cell sheet, which is frozen in the inside of the culture vessel or the cryopreservation vessel, can be a package packaged with a packaging material in a state of being frozen in the culture vessel or the cryopreservation vessel or in a state of being taken out from the container. By sealing the package, the cell sheet can be stored and circulated without being contaminated with microorganisms and without being damaged. As an example, by freezing a container having the culturing carrier substrate with a cell sheet and the cryopreservation solution in a state of being packaged and sealed with a film-like packaging material, a package of the frozen product can be obtained. As the packaging material, aluminum, polyethylene terephthalate, ionomer, polyethylene, polyvinylidene chloride, polyvinyl alcohol, polypropylene, polyester, polycarbonate, polyacrylonitrile, ethylene-vinyl acetate copolymer, ethylene-vinyl alcohol copolymer, ethylene-methacrylic acid copolymer, polyimide, fluororesin such as perfluoroalkoxy fluororesin, tetrafluoroethylene-hexafluoropropylene copolymer (FEP), or nylon is preferable. These may be used alone or in combination of two or more thereof.

### (Thawing Step)

In a case where the transplantation method according to the present embodiment includes the cryopreservation method, the cryopreservation method further includes a thawing step of thawing the culturing carrier substrate with a cell sheet 50.

The thawing method is not particularly limited, and a usual method used in the technical field of medicine, pharmaceuticals, cosmetics, food, veterinary drugs, and the like, and the basic technology field of regenerative medicine, bioengineering, and the like can be used.

Examples of the thawing method include the use of a water bath, an incubator, a hot plate, a defroster, and the like, and immersing the frozen product in a thawing solution at a temperature higher than the freezing temperature, and standing the frozen product in a temperature environment higher than the freezing temperature.

An environmental temperature in contact with the frozen product during thawing is not particularly limited as long as it is a temperature higher than the freezing temperature and lower than 50°C. The upper limit of the environmental temperature is, for example, equal to or lower than 49°C, preferably equal to or lower than 45°C, and more preferably equal to or lower than 40°C. On the other hand, the lower limit of the environmental temperature is, for example, equal to or higher than 0°C, preferably equal to or higher than 5°C, more preferably equal to or higher than 10°C, and still more preferably equal to or higher than 15°C.

The range of the environmental temperature in contact with the frozen product during thawing is, for example, equal to or higher than 0°C and equal to or lower than 49°C, equal to or higher than 0°C and equal to or lower than 45°C, equal to or higher than 0°C and equal to or lower than 40°C, equal to or higher than 5°C and equal to or lower than 49°C, equal to or higher than 5°C and equal to or lower than 45°C, equal to or higher than 5°C and equal to or lower than 40°C, equal to or higher than 10°C and equal to or lower than 49°C, equal to or higher than 10°C and equal to or lower than 45°C, equal to or higher than 10°C and equal to or lower than 40°C, equal to or higher than 15°C and equal to or lower than 49°C, equal to or higher than 15°C and equal to or lower than 45°C, or equal to or higher than 15°C and equal to or lower than 40°C.

In a case where the thawing temperature is equal to or higher than 50°C, the cell may be damaged by heat, which is not preferable. In addition, the frozen product may be temporarily stored in an environment at a temperature equal to or lower than the freezing point during thawing. For example, the frozen product stored at -80°C can be thawed in an environment at a temperature equal to or higher than the freezing point after being exposed to an environmental temperature of -30°C.

The time required for thawing the frozen product is not particularly limited as long as the cell is not damaged by freezing. In general, the frozen product can be thawed without any problem as long as the time is more than 10 seconds and equal to or less than 60 minutes. The lower limit value of the time required for thawing the frozen product need only be more than 10 seconds, and is preferably equal to or more than 20 seconds, more preferably equal to or more than 30 seconds, and still more preferably equal to or more than 1 minute. The upper limit value thereof need only be equal to or less than 60 minutes, and is preferably equal to or less than 50 minutes, more preferably equal to or less than 40 minutes, and still more preferably equal to or less than 30 minutes. For example, the time required for thawing is more than 10 seconds and equal to or less than 60 minutes, more than 10 seconds and equal to or less than 50 minutes, more than 10 seconds and equal to or less than 40 minutes, more than 10 seconds and equal to or less than 30 minutes, equal to or more than 20 seconds and equal to or less than 60 minutes, equal to or more than 20 seconds and equal to or less than 50 minutes, equal to or more than 20 seconds and equal to or less than 40 minutes, equal to or more than 20 seconds and equal to or less than 30 minutes, equal to or more than 30 seconds and equal to or less than 60 minutes, equal to or more than 30 seconds and equal to or less than 50 minutes, equal to or more than 30 seconds and equal to or less than 40 minutes, equal to or more than 30 seconds and equal to or less than 30 minutes, equal to or more than 1 minute and equal to or less than 60 minutes, equal to or more than 1 minute and equal to or less than 50 minutes, equal to or more than 1 minute and equal to or less than 40 minutes, or equal to or more than 1 minute and equal to or less than 30 minutes.

In a case where the thawing is too fast, the frozen product may crack due to thermal shock caused by a temperature difference, and the cell sheet may be damaged. In a case where the thawing is too slow, water molecules recrystallize under a freezing point condition, ice crystals grow, and the cell is severely damaged, which is not preferable.

During the above-described thawing time, the above-described environmental temperature may be set to be constant, or may be set to change stepwise or the like.

The thawing solution is not particularly limited as long as it does not damage the culture cells. Examples of the component contained in the thawing solution include sucrose, glucose, maltose, trehalose, and fructose. In addition, the thawing solution may contain the components described in the above (culture liquid).

The temperature of the thawing solution is not particularly limited as long as it is a temperature higher than the freezing temperature. The temperature of the thawing solution is, for example, equal to or higher than 0°C and equal to or lower than 45°C. The lower limit value thereof is more preferably equal to or higher than 4°C, still more preferably equal to or higher than 25°C, and equal to or higher than particularly preferably 28°C. On the other hand, the upper limit value thereof is more preferably equal to or lower than 40°C, still more preferably equal to or lower than 39°C, and particularly preferably equal to or lower than 38°C. The temperature of the thawing solution is, for example, equal to or higher than 0°C and equal to or lower than 45°C, equal to or higher than 0°C and equal to or lower than 40°C, equal to or higher than 0°C and equal to or lower than 39°C, equal to or higher than 0°C and equal to or lower than 38°C, equal to or higher than 4°C and equal to or lower than 45°C, equal to or higher than 4°C and equal to or lower than 40°C, equal to or higher than 4°C and equal to or lower than 39°C, equal to or higher than 4°C and equal to or lower than 38°C, equal to or higher than 25°C and equal to or lower than 45°C, equal to or higher than 25°C and equal to or lower than 40°C, equal to or higher than 25°C and equal to or lower than 39°C, equal to or higher than 25°C and equal to or lower than 38°C, equal to or higher than 28°C and equal to or lower than 45°C, equal to or higher than 28°C and equal to or lower than 40°C, equal to or higher than 28°C and equal to or lower than 39°C, or equal to or higher than 28°C and equal to or lower than 38°C.

The thawed cell sheet 40 and culturing carrier substrate 10 (culturing carrier substrate with a cell sheet 50) may be washed with a cell washing solution immediately after the thawing treatment, as necessary. The cell washing solution is not particularly limited, and may contain the components described in the above (culture liquid).

The temperature of the cell washing solution is not particularly limited. The temperature of the cell washing solution is, for example, equal to or higher than 0°C and equal to or lower than 45°C. The lower limit value thereof is more preferably equal to or higher than 4°C, still more preferably equal to or higher than 25°C, and equal to or higher than particularly preferably 28°C. On the other hand, the upper limit value thereof is more preferably equal to or lower than 40°C, still more preferably equal to or lower than 39°C, and particularly preferably equal to or lower than 38°C.

The number of times of washing the culture cells is not particularly limited, and is once or a plurality of times (for example, twice, three times, four times, five times, and the like).

In the present disclosure, since the culturing surface 12 does not contain the temperature-responsive polymer, the cell sheet 40 can be suppressed from being peeled off from the culturing carrier substrate 10 in a low-temperature environment such as the cryopreservation step, and the culturing carrier substrate with a cell sheet 50 in which the adhesiveness between the cell sheet and the film is maintained can be obtained even after the cryopreservation step and the thawing step.

Although the embodiments of the present invention have been described above, these are examples of the present invention, and various configurations other than the above can be adopted. In addition, the present invention is not limited to the embodiments described above and modifications, improvements, and the like are included in the present invention in a range in which it is possible to achieve the object of the present invention.

Hereinafter, examples of reference embodiments A to J will be added.

### <A. Culturing Carrier Substrate>

Examples of the reference embodiment A of the culturing carrier substrate include the following 1. to 13.
1. A culturing carrier substrate used for transporting a cell sheet cultured over a culturing surface,
   in which the culturing carrier substrate has a substrate thickness equal to or more than 5 µm and equal to or less than 250 µm.
2. The culturing carrier substrate according to 1.,
   in which the substrate thickness of the culturing carrier substrate is more than 10 µm, and the culturing carrier substrate functions as a self-supporting film without being fixed to an inside of a culture vessel during culturing of the cell sheet.
3. The culturing carrier substrate according to 1. or 2.,
   in which the culturing surface is subjected to a hydrophilization treatment.
4. The culturing carrier substrate according to 3.,
   in which the hydrophilization treatment is a UV ozone treatment or a plasma treatment.
5. The culturing carrier substrate according to any one of 1. to 4.,
   in which the culturing carrier substrate is a polyether ether ketone film or a polyethylene terephthalate film.
6. The culturing carrier substrate according to any one of 1. to 5.,
   in which the culturing surface does not contain a temperature-responsive polymer.
7. The culturing carrier substrate according to any one of 1. to 6.,
   in which an area of the culturing carrier substrate is equal to or more than 0.3 cm² and equal to or less than 1000 cm².
8. The culturing carrier substrate according to any one of 1. to 7.,
   in which the culturing carrier substrate includes one or two or more resin layers consisting of a resin material.
9. The culturing carrier substrate according to any one of 1. to 8.,
   in which, in a case where the culturing surface is measured with a laser microscope, there are three or less holes having a diameter equal to or more than 1 µm and equal to or less than 100 µm and a depth equal to or more than 0.5 µm and equal to or less than 100 µm in at least one of an observation range of 100 µm square.
10. The culturing carrier substrate according to any one of 1. to 9.,
   in which a void ratio is equal to or less than 15%.
11. The culturing carrier substrate according to any one of 1. to 10.,
   in which a water contact angle on the culturing surface is equal to or less than 70°.
12. The culturing carrier substrate according to any one of 1. to 11.,
   in which the culturing carrier substrate is used for cryopreserving a cell sheet formed on the culturing surface.
13. The culturing carrier substrate according to any one of 1. to 12.,
   in which the culturing carrier substrate is used for attaching a cell sheet formed on the culturing surface to a transplantation site.

### <B. Culturing Carrier Substrate with Cell Sheet>

Examples of the reference embodiment B of the culturing carrier substrate with a cell sheet include the following 1. to 21.
1. A culturing carrier substrate with a cell sheet, including:
   a laminate of a culturing carrier substrate and a cell sheet,
   in which the cell sheet is in a state of covering at least 50% of a surface of the culturing carrier substrate on a culturing surface side.
2. The culturing carrier substrate with a cell sheet according to 1.,
   in which a substrate thickness of the culturing carrier substrate is equal to or more than 5 µm and equal to or less than 250 µm.
3. The culturing carrier substrate with a cell sheet according to 1. or 2.,
   in which the culturing surface is subjected to a hydrophilization treatment.
4. The culturing carrier substrate with a cell sheet according to 3.,
   in which the hydrophilization treatment is a UV ozone treatment or a plasma treatment.
5. The culturing carrier substrate with a cell sheet according to any one of 1. to 4.,
   in which the culturing carrier substrate is a polyether ether ketone film or a polyethylene terephthalate film.
6. The culturing carrier substrate with a cell sheet according to any one of 1. to 5.,
   in which the culturing surface does not contain a temperature-responsive polymer.
7. The culturing carrier substrate with a cell sheet according to any one of 1. to 6.,
   in which an area of the culturing carrier substrate is equal to or more than 0.3 cm² and equal to or less than 1000 cm².
8. The culturing carrier substrate with a cell sheet according to any one of 1. to 7.,
   in which the culturing carrier substrate includes one or two or more resin layers consisting of a resin material.
9. The culturing carrier substrate with a cell sheet according to any one of 1. to 8.,
   in which, in a case where the culturing surface is measured with a laser microscope, there are three or less holes having a diameter equal to or more than 1 µm and equal to or less than 100 µm and a depth equal to or more than 0.5 µm and equal to or less than 100 µm in at least one of an observation range of 100 µm square.
10. The culturing carrier substrate with a cell sheet according to any one of 1. to 9.,
   in which a void ratio is equal to or less than 15%.
11. The culturing carrier substrate with a cell sheet according to any one of 1. to 10.,
   in which a water contact angle on the culturing surface is equal to or less than 70°.
12. The culturing carrier substrate with a cell sheet according to any one of 1. to 11.,
   in which the culturing carrier substrate is used for transporting a cell sheet cultured on a culturing surface.
13. The culturing carrier substrate with a cell sheet according to any one of 1. to 12.,
   in which the culturing carrier substrate is used for cryopreserving a cell sheet formed on the culturing surface.
14. The culturing carrier substrate with a cell sheet according to any one of 1. to 13.,
   in which the culturing carrier substrate is used for attaching a cell sheet formed on the culturing surface to a transplantation site.
15. The culturing carrier substrate with a cell sheet according to any one of 1. to 14.,
   in which the culturing carrier substrate with a cell sheet is used for a cell transplantation therapy.
16. The culturing carrier substrate with a cell sheet according to any one of 1. to 15.,
   in which the cell transplantation therapy includes at least one of suppressing or preventing the onset and relapse of symptoms related to the deficiency and functional disorders, and functional failure of cells, tissues, and organs.
17. The culturing carrier substrate with a cell sheet according to any one of 1. to 16.,
   in which the culturing carrier substrate with a cell sheet is used for treating at least one of spinal cord injury, knee joint cartilage injury, ischemic heart disease, age-related macular degeneration, corneal epithelial stem cell deficiency, aplastic anemia, severe lower limb ischemia, intractable skin ulcer, prevention of postoperative complications, or burn.
18. The culturing carrier substrate with a cell sheet according to any one of 1. to 17.,
   in which the culturing carrier substrate with a cell sheet is used for a transplantation method including at least one of a culturing step of a cell sheet, a cryopreservation step of a cell sheet, a thawing step of a frozen product of a cell sheet, or an attachment step of a cell sheet to a transplantation site.
19. A transplant material including: the culturing carrier substrate with a cell sheet according to any one of 1. to 18.
20. The transplant material according to 19.,
   in which the transplant material is used for attaching a cell sheet to a disease site.
21. The transplant material according to 19. or 20.,
   in which the transplant material is a transplant material for treatment for treating at least one of spinal cord injury, knee joint cartilage injury, ischemic heart disease, age-related macular degeneration, corneal epithelial stem cell deficiency, aplastic anemia, severe lower limb ischemia, intractable skin ulcer, prevention of postoperative complications, or burn.

The culturing carrier substrate with a cell sheet may include any one of the culturing carrier substrates of 1 to 13 of the reference embodiment A.

### <C. Frozen Product of Culturing Carrier Substrate with Cell Sheet>

Examples of the reference embodiment C of the frozen product of the culturing carrier substrate with a cell sheet include the following 1. to 8.
1. A frozen product including: a culturing carrier substrate; and a cell sheet formed on a surface of the culturing carrier substrate,
   in which the culturing carrier substrate and the cell sheet are in a cryopreserved state.
2. The frozen product according to 1.,
   in which the frozen product is in a cryopreserved state at -196°C to -60°C.
3. The frozen product according to 1.,
   in which the frozen product is in a cryopreserved state at -196°C to -135°C.
4. The frozen product according to 1.,
   in which the frozen product is in a cryopreserved state at -134°C to -60°C.
5. The frozen product according to any one of 1 to 4, further including: a cryopreservation solution.
6. The frozen product according to 5,
   in which the cryopreservation solution includes at least one of a culture liquid component or a freezing protective agent.
7. The frozen product according to 5. or 6.,
   in which the cryopreservation solution does not contain DMSO.
8. The frozen product according to any one of 1. to 4.,
   in which an entire part of the culturing carrier substrate and the cell sheet is in a cryopreserved state in which the culturing carrier substrate and the cell sheet are covered with a cryopreservation solution.

In the frozen product of the culturing carrier substrate with a cell sheet, any one of the culturing carrier substrates of 1. to 13. of the reference embodiment A may be included, or any one of the culturing carrier substrates with a cell sheet of 1. to 21. of the reference embodiment B may be included.

### <D. Package>

Examples of the reference embodiment D of the package include the following 1. to 8.
1. A package including: a culturing carrier substrate; and a packaging material that packages the culturing carrier substrate.
2. A package including:
   a culturing carrier substrate with a cell sheet, the culturing carrier substrate with a cell sheet including a culturing carrier substrate and a cell sheet formed on a culturing surface of the culturing carrier substrate; and
   a packaging material that packages the culturing carrier substrate with a cell sheet.
3. A package comprising:
   a frozen product of a culturing carrier substrate with a cell sheet, the culturing carrier substrate with a cell sheet including a culturing carrier substrate and a cell sheet formed on a culturing surface of the culturing carrier substrate;
   and a packaging material that packages the frozen product.
4. The package according to any one of 1. to 3.,
   in which an inside of the packaging material includes a culture vessel and/or a cryopreservation vessel.
5. The package according to any one of 1. to 4.,
   in which an inside of the packaging material is in a sealed state.
6. The package according to any one of 1. to 5.,
   in which the package is used for transport and/or storage.

In the package, any one of the culturing carrier substrates of 1. to 13. of the reference embodiment A may be included, any one of the culturing carrier substrates with a cell sheet of 1. to 21. of the reference embodiment B may be included, or any one of the frozen products of the culturing carrier substrates with a cell sheet of 1. to 8. of the reference embodiment C may be included.

### <E. Cryopreservation Method or Thawing Method>

Examples of the reference embodiment E of the cryopreservation method or the thawing method include the following 1. to 10.
1. A cryopreservation method including: a cooling step of cooling a culturing carrier substrate with a cell sheet, the culturing carrier substrate with a cell sheet including a culturing carrier substrate and a cell sheet formed on a surface of the culturing carrier substrate, in a cryopreservation solution.
2. The cryopreservation method according to 1.,
   in which in the cooling step, the culturing carrier substrate with a cell sheet is cooled in the cryopreservation solution using a non-flow-through-type cooling device.
3. The cryopreservation method according to 1. or 2.,
   in which in the cooling step, a cooling rate at 0°C to -5°C is equal to or more than 0.1°C/min and equal to or less than 15°C/min.
4. The cryopreservation method according to any one of 1. to 3., further including:
   a cryopreservation step of storing the culturing carrier substrate with a cell sheet at -196°C to -60°C after the cooling step.
5. The cryopreservation method according to any one of 1. to 4.,
   in which the frozen product including the culturing carrier substrate and the cell sheet is obtained by the cooling step.
6. A thawing method including:
   a thawing step of thawing a frozen product including a culturing carrier substrate and a cell sheet formed on a surface of the culturing carrier substrate, the culturing carrier substrate and the cell sheet being in a cryopreserved state.
7. The thawing method according to 6.,
   in which the thawing step is included after the cooling step of the cryopreservation method according to any one of 1. to 4.
8. The thawing method according to 6. or 7., further including:
   a thawing step of thawing the frozen product obtained by the cryopreservation method according to 5.
9. The thawing method according to any one of 6. to 8.,
   in which in the thawing step, an environmental temperature during thawing is equal to or higher than 0°C and lower than 50°C.
10. The thawing method according to any one of 6. to 9.,
   in which in the thawing step, a thawing time is more than 10 seconds and equal to or less than 60 minutes.

In the cryopreservation method or the freezing method, any one of the culturing carrier substrates of 1. to 13. of the reference embodiment A may be included, or any one of the culturing carrier substrates with a cell sheet of 1. to 21. of the reference embodiment B may be included.

The frozen product obtained by the cryopreservation method may include any one of the frozen products of the culturing carrier substrates with a cell sheet of 1. to 8. of the reference embodiment C.

In addition, the frozen product obtained by the cryopreservation method and/or the thawed product obtained by the thawing method can be used for a transplantation method described below.

### <F. Method of Manufacturing Culturing Carrier Substrate with Cell Sheet>

Examples of the reference embodiment F of the method of manufacturing a culturing carrier substrate with a cell sheet include the following 1. to 3.
1. A method of manufacturing a culturing carrier substrate with a cell sheet, the method including:
   a culturing step of introducing a plurality of cells, a culture medium, and a culturing carrier substrate into an inside of a culture vessel to culture a cell sheet on a culturing surface of the culturing carrier substrate; and
   an extraction step of extracting the culturing carrier substrate with the cell sheet from the culture vessel.
2. The method of manufacturing a culturing carrier substrate with a cell sheet according to 1.,
   in which in the culturing step, the cell sheet is cultured without fixing the culturing carrier substrate to an inside of a culture vessel.
3. The method of manufacturing a culturing carrier substrate with a cell sheet according to 1. or 2.,
   in which in the extraction step, an operation of separating the culturing carrier substrate from the culture vessel by a physical operation is not required.

In the method of manufacturing a culturing carrier substrate with a cell sheet, any one of the culturing carrier substrates of 1. to 13. of the reference embodiment A may be included.

### <G. Transplantation Method>

Examples of the reference embodiment G of the transplantation method include the following 1. to 25.
1. A transplantation method including: a transplantation step of attaching a cell sheet to a transplantation site using a culturing carrier substrate with a cell sheet, the culturing carrier substrate with a cell sheet including a culturing carrier substrate and a cell sheet formed on a culturing surface of the culturing carrier substrate, and then peeling off the culturing carrier substrate from the cell sheet.
2. The transplantation method according to 1.,
   in which in the transplantation step, a culturing carrier substrate with a cell sheet, the culturing carrier substrate with a cell sheet having a laminate of the culturing carrier substrate and the cell sheet and in which the cell sheet is in a state of covering at least 50% of a surface of the culturing carrier substrate on a culturing surface side, is used.
3. The transplantation method according to 1. or 2.,
   in which in the transplantation step, the culturing carrier substrate with a cell sheet is taken out from a package and used, or a frozen product of the culturing carrier substrate with a cell sheet is taken out from a package and thawed and then used.
4. The transplantation method according to any one of 1. to 3., further including:
   a culturing step of culturing the cell sheet on the culturing surface of the culturing carrier substrate to obtain the culturing carrier substrate with a cell sheet.
5. The transplantation method according to 4.,
   in which in the culturing step, the cell sheet is cultured in a state in which at least a part of each of the culturing surface and a side surface of the culturing carrier substrate is brought into contact with a culture liquid.
6. The transplantation method according to 4. or 5.,
   in which in the culturing step, the cell sheet is cultured in a state in which the culturing carrier substrate is fixed by a weight and/or the culturing carrier substrate is fixed in position by an instrument.
7. The transplantation method according to any one of 4. to 6.,
   in which in the culturing step, a density of the cells to be cultured is equal to or more than 5 × 10² cells/cm² and equal to or less than 1 × 10⁹ cells/cm².
8. The transplantation method according to any one of 4. to 7.,
   in which a substrate thickness of the culturing carrier substrate is more than 10 µm, and in the culturing step, the culturing carrier substrate functions as a self-supporting film without being fixed to an inside of a culture vessel.
9. The transplantation method according to any one of 1. to 8., further including:
   a cooling and storage step of cooling the culturing carrier substrate with a cell sheet in a cryopreservation solution to obtain a frozen product of the culturing carrier substrate with a cell sheet.
10. The transplantation method according to any one of 1. to 9., further including:
   a thawing step of thawing a frozen product of the culturing carrier substrate with a cell sheet.
11. The transplantation method according to any one of 1. to 10.,
   in which a substrate thickness of the culturing carrier substrate is equal to or more than 5 µm and equal to or less than 250 µm,
   the culturing carrier substrate is a polyether ether ketone film or a polyethylene terephthalate film, and
   the culturing surface of the culturing carrier substrate is subjected to a hydrophilization treatment.
12. The transplantation method according to any one of 1. to 11.,
   in which in the transplantation step, a time from the attachment to the peeling off is equal to or less than 10 minutes.
13. The transplantation method according to any one of 1. to 12.,
   in which an area of the cell sheet is equal to or more than 0.3 cm².
14. The transplantation method according to any one of 1. to 13.,
   in which after the transplantation step, a remaining ratio of the cell sheet on the culturing carrier substrate after the peeling is equal to or less than 50% in terms of area ratio.
15. The transplantation method according to any one of 1. to 14.,
   in which a thickness of the cell sheet is equal to or more than 0.001 mm and equal to or less than 2.0 mm.
16. The transplantation method according to any one of 1. to 15.,
   in which an area of the culturing carrier substrate is equal to or more than 0.3 cm² and equal to or less than 1000 cm².
17. The transplantation method according to any one of 1. to 16.,
   in which the culturing carrier substrate includes one or two or more resin layers consisting of a resin material.
18. The transplantation method according to any one of 1. to 17.,
   in which a culturing carrier substrate in which, in a case where the culturing surface is measured with a laser microscope, there are three or less holes having a diameter equal to or more than 1 µm and equal to or less than 100 µm and a depth equal to or more than 0.5 µm and equal to or less than 100 µm in at least one of an observation range of 100 µm square is used.
19. The transplantation method according to any one of 1. to 18.,
   in which a void ratio of the culturing carrier substrate is equal to or less than 15%.
20. The transplantation method according to any one of 1. to 19.,
   in which a water contact angle on the culturing surface of the culturing carrier substrate is equal to or less than 70°.
21. The transplantation method according to any one of 1. to 20.,
   in which a culturing carrier substrate with a cell sheet in which the cell sheet is in a state of covering equal to or more than 50% and equal to or less than 100% of a region in which the cells can be deposited on the culturing surface of the culturing carrier substrate is obtained.
22. The transplantation method according to any one of 1. to 21.,
   in which the transplantation method is used for a cell transplantation therapy.
23. The transplantation method according to 22.,
   in which the cell transplantation therapy includes at least one of suppressing or preventing the onset and relapse of symptoms related to the deficiency and functional disorders, and functional failure of cells, tissues, and organs.
24. The transplantation method according to any one of 1. to 23.,
   in which in the transplantation step, the cell sheet is attached to a disease site.
25. The transplantation method according to any one of 1. to 24.,
   in which the disease includes at least one of spinal cord injury, knee joint cartilage injury, ischemic heart disease, age-related macular degeneration, corneal epithelial stem cell deficiency, aplastic anemia, severe lower limb ischemia, intractable skin ulcer, prevention of postoperative complications, or burn.

In the transplantation method, any one of the culturing carrier substrates of 1. to 13. of the reference embodiment A may be included, any one of the culturing carrier substrates with a cell sheet of 1. to 21. of the reference embodiment B may be included, or any one of the frozen products of the culturing carrier substrates with a cell sheet of 1. to 8. of the reference embodiment C may be included.

In addition, in the transplantation method, any one of the methods of manufacturing a culturing carrier substrate with a cell sheet of 1. to 3. of the reference embodiment F or the culturing step thereof may be included, or any one of the cryopreservation methods or the thawing methods of 1. to 10. of the reference embodiment E may be included.

In addition, examples of the reference embodiment H include a treatment method including at least one of suppressing or preventing the onset and relapse of symptoms related to the deficiency and functional disorders, and functional failure of cells, tissues, and organs, using at least one of any one of the culturing carrier substrates of 1. to 13. of the reference embodiment A, any one of the culturing carrier substrates with a cell sheet of 1. to 21. of the reference embodiment B, or any one of the frozen products of the culturing carrier substrates with a cell sheet of 1. to 8. of the reference embodiment C. In this treatment method, the related symptoms may include at least one of the above-described diseases.

In addition, examples of the reference embodiment I include a use of at least one of any one of the culturing carrier substrates of 1. to 13. of the reference embodiment A, any one of the culturing carrier substrates with a cell sheet of 1. to 21. of the reference embodiment B, or any one of the frozen products of the culturing carrier substrates with a cell sheet of 1. to 8. of the reference embodiment C for treatment including at least one of suppressing or preventing the onset and relapse of symptoms related to the deficiency and functional disorders, and functional failure of cells, tissues, and organs. In this use, the related symptoms may include at least one of the above-described diseases.

In addition, examples of the reference embodiment J include a use of at least one of any one of the culturing carrier substrates of 1. to 13. of the reference embodiment A, any one of the culturing carrier substrates with a cell sheet of 1. to 21. of the reference embodiment B, or any one of the frozen products of the culturing carrier substrates with a cell sheet of 1. to 8. of the reference embodiment C in the manufacture of a pharmaceutical for treatment including at least one of suppressing or preventing the onset and relapse of symptoms related to the deficiency and functional disorders, and functional failure of cells, tissues, and organs. In this use, the related symptoms may include at least one of the above-described diseases.

### Examples

A detailed description will be given below of the present invention with reference to Examples, but the present invention is not limited to the descriptions of these Examples.

### [Example 1]

### <Manufacturing of Culturing Carrier Substrate>

The culturing surface (surface) of the PEEK film (manufactured by Shin-Etsu Polymer Co., Ltd., polyether ether ketone film, Shin-Etsu Sepla Film (registered trademark) low crystalline type 12 µm thickness) was subjected to a plasma treatment. The surface roughness after the plasma treatment was roughened as compared with that before the plasma treatment, and the Ra thereof was 0.6 nm. The Ra was measured as follows. Using an AFM (manufactured by Shimadzu Corporation, SPM-9700), a silicon single crystal probe (curvature radius of about 10 nm) having a small tip diameter was used as a probe. The surface was observed in a range of 1 µm × 1 µm. For the calculation of the Ra, analysis software attached to the device was used, and the surface roughness analysis was executed in a range of 100 nm × 100 nm avoiding foreign substances and scratches on the surface after the smoothing processing in the X and Y directions. The arithmetic average roughness Ra is an average value of distances from a reference line within a reference length.

In addition, the PEEK film had a void ratio equal to or less than 5% and was a solid film in which there was no hole having a diameter equal to or more than 1 µm and equal to or less than 100 µm and a depth equal to or more than 0.5 µm and 100 µm in a range of 100 µm square in a case where the culturing surface was measured with a laser microscope. The void ratio was calculated from "{1 - (measured density/theoretical density)} × 100".

Thereafter, the obtained surface-treated film was cut into a size of Φ33.5 mm to obtain a disk-shaped culturing carrier substrate.

### <Production of Cell Sheet>

The culturing carrier substrate manufactured in the above <Manufacturing of Culturing Carrier Substrate> was washed with 70% ethanol, a phosphate buffer solution, and a culture medium in this order, and then placed in each flat bottom of the holes of the cell culture multi-well plate (6 wells). In this case, the culturing surface of the culturing carrier substrate was disposed to face the opening of the well plate, that is, the back surface of the culturing carrier substrate was in contact with the flat bottom of the hole portion of the well plate. However, the culturing carrier substrate and the inside of the well plate (culture vessel) were not fixed to each other.

The cryopreserved human fibroblasts (derived from human oral tissue) were thawed at 37°C and washed with a culture medium. 5 × 10⁵ cells were suspended in a culture medium containing 5% serum and seeded at 2.5 × 10⁵ cells on each of two dishes (60.1 cm²), and then cultured for 3 days. The cultured cells were recovered, suspended in a culture medium containing 5% serum, seeded at 2.5 × 10⁵ cells on each of four flasks (225 cm²), and subcultured, followed by culturing for four days.

The cultured cells were recovered and suspended in a culture medium containing 2% serum. The cell suspension was seeded at a seeding density of 55.8 × 10⁴ cells/cm² in each well of the cell culture multi-well plate with the culturing carrier substrate installed, and incubated at 37°C and 5% CO₂ for 1 day to form a cell sheet on the culturing surface of the culturing carrier substrate.

### <Transplant>

After the above <Production of Cell Sheet>, the culturing carrier substrate with a cell sheet was taken out from the multiplate. The taken-out culturing carrier substrate with a cell sheet was transported to a pork slice (transplantation site) imitating a cell tissue prepared at another place. The surface of the cell sheet was attached to the surface of the pork slice, and then the culturing carrier substrate was peeled off from the cell sheet.

It is noted that, since the substrate was not particularly cooled during the period from the attachment to the peeling, the temperature of the substrate was maintained at a temperature equal to or higher than 30°C.

### [Example 2]

In the above <Manufacturing of culturing carrier substrate>, a PET film (manufactured by Toray Industries, Inc., polyethylene terephthalate film, LUMIRROR (registered trademark)) having a thickness of 25 µm was used instead of the polyether ether ketone film, and <Manufacturing of culturing carrier substrate>, <Production of Cell Sheet>, and <Transplant> were performed in the same manner as in Example 1.

### [Example 3]

In the above <Manufacturing of culturing carrier substrate>, the cutting size of the surface-treated film was set to Φ14.0 mm instead of Φ33.5 mm, and in the above <Production of Cell Sheet>, the use of the 24-hole flat-bottom cell culture multi-well plate and the seeding density were changed to 27.9 × 10⁴ cells/cm² in a case of seeding, and <Production of Cell Sheet> and <Transplant> were performed in the same manner as in Example 1.

### [Comparative Example 1]

In <Production of Cell Sheet>, a cell sheet was produced on a flat bottom in a hole of a multi-well plate for adherent cells, and in <Transplant>, the cell sheet was peeled off from the flat bottom and attached to the surface of the pork slice, without using the culturing carrier substrate manufactured in the above <Manufacturing of culturing carrier substrate>, and <Production of Cell Sheet> and <Transplant> were performed in the same manner as in Example 1.

### [Comparative Example 2]

In the above <Manufacturing of culturing carrier substrate>, the plasma treatment was not performed on the culturing surface of the substrate, and <Manufacturing of culturing carrier substrate>, <Production of Cell Sheet>, and <Transplant> were performed in the same manner as in Example 1.

### [Comparative Example 3]

In the above <Manufacturing of culturing carrier substrate>, the plasma treatment was not performed on the culturing surface of the substrate, and <Manufacturing of culturing carrier substrate>, <Production of Cell Sheet>, and <Transplant> were performed in the same manner as in Example 2.

The culture and the transplantation of the cell sheet were evaluated for each of Examples and Comparative Examples. The results are listed in Table 1.

### [Table 1]

**Table 1**

| | Culturing Carrier Substrate | | Evaluation | |
|---|---|---|---|---|
| | Material of Substrate | Surface Treatment | Cell Sheet Culture | Transplantation |
| Example 1 | PEEK | Plasma-treated | Good | Good |
| Example 2 | PET | Plasma-treated | Good | Good |
| Example 3 | PEEK | Plasma-treated | Good | Good |
| Comparative Example 1 | 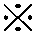 Not used | | Good | Poor |
| Comparative Example 2 | PEEK | Not plasma-treated | Poor | - |
| Comparative Example 3 | PET | Not plasma-treated | Poor | - |

In Examples 1 to 3, in <Production of Cell Sheet>, a culturing carrier substrate with a cell sheet in which the cell sheet covered equal to or more than 100% of the substrate culturing surface could be obtained.

In <Transplant>, since the culturing carrier substrate with a cell sheet was not fixed to the multiplate, it could be easily taken out. It was found that, in the PET film of Example 2, the substrate was peeled off by slightly pressing the cell sheet with forceps, but in the PEEK films of Examples 1 and 3, the substrate could be more easily peeled off by sliding the substrate without pressing the cell sheet, and the handleability was excellent.

In addition, in <Transplant>, the culturing carrier substrate was easily taken off from the cell sheet. Specifically, since the cell sheet and the pork were quickly adhered to each other, the attachment to the peeling was performed within 1 minute. That is, since the operation at the time of transplantation is simple, the operation time can be shortened. It is noted that, after <Transplant>, it was not necessary to culture the cell sheet on the pork.

From the results of Examples 1 to 3, it was confirmed that both the culture and the transplantation of the cell sheet could be performed by using the plasma-treated PEEK film or the plasma-treated PET film as the culturing carrier substrate.

In addition, in <Transplant> of Examples 1 to 3, the residual rate of the cell sheet remaining on the culturing surface of the culturing carrier substrate after the peeling was equal to or less than 1% in terms of area ratio. Therefore, it is possible to reduce the cell sheet remaining at the time of the transplantation step. It is noted that, in a case of peeling off the culturing carrier substrate from the cell sheet, it was not necessary to perform a dispase treatment on the culturing surface before the culture.

In Comparative Example 1, in <Production of Cell Sheet>, the cell sheet could be produced in the multi-well plate for adherent cells. However, in <Transplant>, it was difficult to perform an operation of peeling off the cell sheet from the flat bottom of the multi-well plate for adherent cells in order to prevent the cell sheet from being damaged by forceps or the like in a case of taking out the cell sheet from the multi-well plate for adherent cells. In addition, even though the cell sheet could be peeled off, it was difficult to attach the cell sheet because the cell sheet could not be gripped.

In Comparative Examples 2 and 3, since the cell sheet could not be produced, the transplantation operation was not performed.

### [Example 4]

### <Production of Cell Sheet>

A cell sheet (cell sheet with a culturing carrier substrate) was produced on the culturing surface of the culturing carrier substrate using the culturing carrier substrate obtained by setting the cutting size of the surface-treated film to Φ14.0 mm instead of Φ33.5 mm in <Manufacturing of culturing carrier substrate> of Example 1, in the same manner as in <Production of Cell Sheet> of Example 1, except that the use of the 24-hole flat-bottom cell culture multi-well plate and the seeding density were changed to 27.9 × 10⁴ cells/cm² in a case of seeding.

It is noted that the cell viability before freezing was measured according to the following procedure.

### <Cryopreservation of Cell Sheet>

After the above <Production of Cell Sheet>, the cell culture multi-well plate was installed on ice, the cell sheet with a culturing carrier substrate was washed with a phosphate buffer solution, and a freezing protective agent (STEMCELL-BANKER GMP grade, manufactured by Nippon Zenyaku Kogyo Co., Ltd.) was added thereto such that the amount thereof was 0.3 mL/well.

Thereafter, the package in which the cell culture multi-well plate was packaged with a polybag with a chuck was put into a non-flow type cooling device (3D Freezer (registered trademark), KSS-40BLW-2400V, manufactured by KOGASAN Corporation) cooled to -35°C, and held for 30 minutes. The cooling rate at 0°C to -5°C was 3.4°C/min.

Thereafter, the cooled package was quickly put into a -80°C freezer (RDE50086FD type ultra-low temperature freezer, manufactured by Thermo Fisher Scientific Inc.) and held for 1 hour.

In a case where the temperature of the cell sheet (cell sheet with a culturing carrier substrate) stored in the -80°C freezer was measured using a K-type thermocouple, the temperature was -70°C.

### <Thawing of Cell Sheet>

After the freezing period, the package was taken out from the -80°C freezer, and the cell culture multi-well plate in the package was thawed at room temperature in a safety cabinet. The thawing time at this time was 16 minutes. After thawing, the appearance of the cell sheet was good.

After thawing, the freezing protective agent was suctioned, and 2 mL of a culture medium containing 2% serum was added. Thereafter, the cell sheet was incubated at 37°C and 5% CO₂ for 24 hours. After incubation, the cell activity was measured. As a result, it was found that, in a case where the cell activity before freezing was defined as C0 and the cell activity after freezing was defined as C1, the "fluctuation ratio of cell activity before and after freezing" calculated by (C1/C0) × 100 was 89%. In addition, in a case where the culture supernatant was recovered and the measurement of vascular endothelial growth factor (VEGF) and hepatocyte growth factor (HGF) was performed the next day or later after the supernatant was frozen at -80°C, it was found that the VEGF in the supernatant was 1451 pg and the HGF was 4608 pg.

### (Method of Measuring Thawing Time)

The thawing time was a time required for thawing the frozen product cryopreserved by adding the freezing protective agent, and the thawing time was measured from the time of exposure to the environment to be thawed to the time at which the frozen product was completely liquid as an end point.

### (Measurement of Cell Viability)

The cell activity was measured using a cell viability reagent (Cell Count Reagent SF, manufactured by NACALAI TESQUE, INC.) and a plate reader (iMark, manufactured by Bio-Rad Laboratories, Inc.). The cell viability reagent was diluted 20-fold with the culture medium (reagent/culture medium = 1/19 volume ratio) to prepare a test solution. The supernatant of each well of the cell culture multi-well plate containing the cell sheet after culture was suctioned, and 0.5 mL of the test solution was added to each well, and the cell sheet was incubated at 37°C and 5% CO₂ for 1 hour. After incubation, 0.1 mL of the supernatant was added to each well of the cell culture multi-well plate (96 wells, manufactured by Corning Incorporated), and the supernatant was measured with the plate reader. The calculation of the cell activity is as follows.

| | |
|---|---|
| · | Cell activity [Abs.] = {Absorbance of measured sample [Absorbance (450 nm) - Absorbance of measured sample (630 nm)]} -{Absorbance of blank [Absorbance (450 nm) - Absorbance of blank (630 nm)]} |

The blank used the test solution.

### (Measurement of vascular endothelial growth factor (VEGF) and hepatocyte growth factor (HGF))

VEGF and HGF as cytokines involved in angiogenesis were measured. These cytokines were measured using a plate reader (iMark, manufactured by BIO-RAD) with VEGF, Human, ELISA Kit, Quantikine (96 well) (R&D Systems, Inc., DVE00) and HGF, Human, ELISA Kit, Quantikine (96 well) (R&D Systems, Inc., DHG00B). The method of use was used according to the instruction manual of each kit. The test sample was cryopreserved by recovering the supernatant after culturing for 24 hours after thawing. The test sample was thawed at the time of measurement the next day or later after freezing. In addition, the test sample was diluted 2 to 4 times with a diluent attached to the kit, and the measurement was performed. The standard attached to each kit was used for the calibration curve. The absorbance of each sample and the standard for the calibration curve was calculated from the following calculation expression, and the obtained absorbance was fitted to the calibration curve to calculate the concentration of VEGF and HGF.

| | |
|---|---|
| · | Absorbance [Abs.] of sample and standard for calibration curve = Absorbance (450 nm) - Absorbance (570 nm) |

### <Transplantation after Thawing>

The cell sheet with a culturing carrier substrate after thawing was taken out from the cell culture multiplate, the cell sheet surface was attached to the pork, and the substrate was peeled off. It is noted that, since the substrate was not particularly heated or cooled during the period from the attachment to the peeling, the temperature of the substrate was maintained at room temperature (23°C).

### [Examples 5 to 11]

<Production of Cell Sheet>, <Cryopreservation of Cell Sheet>, <Thawing of Cell Sheet>, and <Transplantation after Thawing> were performed in the same manner as in Example 4, except that the freezing period of <Cryopreservation of Cell Sheet> was changed to the value shown in Table 2.

### [Comparative Example 4]

In Comparative Example 4, <Production of Cell Sheet> was performed in the same manner as in Example 4, except that the culturing carrier substrate was not used and the cell culture multi-well plate was changed to a dish (UpCell (registered trademark), manufactured by CellSeed, Inc.).

### [Comparative Example 5]

In Comparative Example 5, <Production of Cell Sheet>, <Cryopreservation of Cell Sheet>, and <Thawing of Cell Sheet> were performed in the same manner as in Example 4, except that the culturing carrier substrate was not used and the cell culture multi-well plate was changed to a multi-well plate for adherent cells (24 holes).

The freezing and storage and the transplantation after thawing were evaluated for each of Examples and Comparative Examples. The results are shown in Table 2.

### [Table 2]

**Table 2**

| | Culturing Carrier Substrate | Freezing Period | Evaluation | | | | |
|---|---|---|---|---|---|---|---|
| | | | Cryopreservation | Fluctuation Ratio of Cell Activity Before/After Freezing (%) | Transplantation After Thawing | VEGF (pg) | HGF (P9) |
| Example 4 | Example 1 | 1 hour | Good | 89 | Good | 1451 | 4608 |
| Example 5 | | 1 day | Good | 116 | Good | 1392 | 4611 |
| Example 6 | | 7 days | Good | 97 | Good | 1293 | 3478 |
| Example 7 | | 14 days | Good | 90 | Good | 1344 | 4088 |
| Example 8 | | 28 days | Good | 94 | Good | 914 | 5292 |
| Example 9 | | 56 days | Good | 81 | Good | 1952 | 5662 |
| Example 10 | | 70 days | Good | 71 | Good | 777 | 4939 |
| Example 11 | | 86 days | Good | 66 | Good | 869 | 4108 |
| Comparative Example 4 | 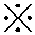 Not used | - | Poor | - | - | - | - |
| Comparative Example 5 | 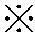 Not used | 86 days | Good | 36 | - | 869 | 3755 |

In Comparative Example 4, since the cell sheet was peeled off from the dish in a case of cooling the dish, the cell sheet could not be cryopreserved. Therefore, the measurement of the cell viability after freezing and the transplantation operation after thawing were not performed.

In Comparative Example 5, the cell sheet could be cryopreserving in a state of being closely adhered to the multi-well plate, but the result was shown that the fluctuation ratio of the cell viability before and after freezing was decreased.

On the other hand, in Examples 4 to 11, the cell sheet could be frozen and stored while being closely adhered to the culturing carrier substrate, and the result was shown that the fluctuation ratio of the cell viability before and after freezing or at least one of VEGF or HGF was higher than that in Comparative Example 5. In addition, in the transplantation after thawing, the culturing carrier substrate could be easily peeled off from the cell sheet. It is noted that, since the cell sheet and the pork were quickly adhered to each other, the attachment to the peeling was performed within 1 minute, and it was not necessary to culture the cell sheet on the pork.

From the results of Examples 4 to 11, it was confirmed that, by using the plasma-treated PEEK film as the culturing carrier substrate, the cell sheet could be frozen and transplanted after thawing, and the cell sheet after thawing could maintain a high cell viability even after being frozen for a long period of time.

### [Examples 12 to 23]

<Production of Cell Sheet>, <Cryopreservation of Cell Sheet>, <Thawing of Cell Sheet>, and <Transplantation after Thawing> were performed in the same manner as in Example 4, except that the freezing period of <Cryopreservation of Cell Sheet> was changed to the value shown in Table 3, Bambanker (registered trademark) hRM (GC LYMPHOTEC Inc.) was used as the freezing protective agent, and a -150°C freezer was used. The thawing time at this time was 18 minutes. After thawing, the appearance of the cell sheet was good.

In a case where the temperature of the cell sheet (cell sheet with a culturing carrier substrate) stored in the -150°C freezer was measured using a K-type thermocouple, the temperature was -140°C.

The freezing and storage and the transplantation after thawing were evaluated for each of Examples. Table 3 shows the results.

### [Table 3]

**Table 3**

| | Culturing Carrier Substrate | Freezing Period | Evaluation | | | | |
|---|---|---|---|---|---|---|---|
| | | | Cryopreservation | Fluctuation Ratio of Cell Activity Before/After Freezing (%) | Transplantation After Thawing | VEGF (pg) | HGF (pg) |
| Example 12 | | 1 day | Good | 117 | Good | 1806 | 3945 |
| Example 13 | | 7 days | Good | 108 | Good | 1794 | 3250 |
| Example 14 | | 14 days | Good | 104 | Good | 1750 | 3600 |
| Example 15 | | 21 days | Good | 129 | Good | 1461 | 3480 |
| Example 16 | | 28 days | Good | 119 | Good | 1416 | 3644 |
| Example 17 | Example 1 | 56 days | Good | 120 | Good | 1754 | 4492 |
| Example 18 | | 70 days | Good | 118 | Good | 1730 | 4250 |
| Example 19 | | 84 days | Good | 114 | Good | 1368 | 5397 |
| Example 20 | | 112 days | Good | 112 | Good | 1748 | 4113 |
| Example 21 | | 168 days | Good | 114 | Good | 2168 | 4573 |
| Example 22 | | 252 days | Good | 113 | Good | 2679 | 3989 |
| Example 23 | | 364 days | Good | 121 | Good | 3019 | 5007 |

In Examples 12 to 23, the cell sheet could be frozen and stored while being closely adhered to the culturing carrier substrate, and the result was shown that the fluctuation ratio of the cell viability before and after freezing or both VEGF and HGF were higher than those in Comparative Example 5. In addition, in the transplantation after thawing, the culturing carrier substrate could be easily peeled off from the cell sheet. It is noted that, since the cell sheet and the pork were quickly adhered to each other, the attachment to the peeling was performed within 1 minute, and it was not necessary to culture the cell sheet on the pork.

### [Examples 24 to 26]

<Production of Cell Sheet>, <Cryopreservation of Cell Sheet>, <Thawing of Cell Sheet>, and <Transplantation after Thawing> were performed in the same manner as in Example 12, except that the freezing period of <Cryopreservation of Cell Sheet> was changed to the value shown in Table 4.

However, the state of freezing in each of Examples was configured as shown in FIGs. 3(A) to 3(C).

In FIG. 3(A), <Cryopreservation of Cell Sheet> and <Thawing of Cell Sheet> were performed in a state where the culturing carrier substrate 10 side was disposed to face the bottom surface of the culture vessel 20, in FIG. 3(B), <Cryopreservation of Cell Sheet> and <Thawing of Cell Sheet> were performed in a state where the cell sheet 40 side was disposed to face the bottom surface of the culture vessel 20, and in FIG. 3(C), <Cryopreservation of Cell Sheet> and <Thawing of Cell Sheet> were performed in a state where the cell sheet 40 was sandwiched between two culturing carrier substrates 10.

The freezing and storage and the transplantation after thawing were evaluated for each of Examples. The results are shown in Table 4.

### [Table 4]

**Table 4**

| | Condition at Time of Freezing | Freezing Period | Evaluation | | |
|---|---|---|---|---|---|
| | | | Cryopreservation | Fluctuation Ratio of Cell Activity Before/After Freezing (%) | Transplantation After Thawing |
| Example 24 | FIG. 3(A) | 6 days | Good | 103 | Good |
| Example 25 | FIG. 3 (B) | 6 days | Good | 96 | Good |
| Example 26 | FIG. 3(C) | 6 days | Good | 100 | Good |

In Examples 24 to 26, the cell sheet could be frozen and stored while being closely adhered to the culturing carrier substrate, and the result was shown that the fluctuation ratio of the cell viability before and after freezing was higher than that in Comparative Example 5. In addition, in the transplantation after thawing, the culturing carrier substrate could be easily peeled off from the cell sheet. It is noted that, since the cell sheet and the pork were quickly adhered to each other, the attachment to the peeling was performed within 1 minute, and it was not necessary to culture the cell sheet on the pork.

It was found that, since the PEEK film had a low coefficient of thermal expansion, the PEEK film could be used as a culturing carrier substrate useful for the freezing treatment.

### [Example 27]

<Production of Cell Sheet>, <Cryopreservation of Cell Sheet>, <Thawing of Cell Sheet>, and <Transplantation after Thawing> were performed in the same manner as in Example 12, except that, in the method shown in <Thawing of Cell Sheet> of Example 12, the thawing at room temperature in the safety cabinet was performed by installing the cell culture multi-well plate on a heat sink of a defroster (manufactured by DEC Co., Ltd.). The set temperature of the defroster indicated the temperature of the heat sink, and the thawing was performed by setting the temperature to 37°C. The thawing time at this time was 3 minutes.

In a case where the temperature of the cell sheet (cell sheet with a culturing carrier substrate) stored in the -150°C freezer was measured using a K-type thermocouple, the temperature was -140°C. After thawing, the appearance of the cell sheet was good.

### [Example 28]

<Production of Cell Sheet>, <Cryopreservation of Cell Sheet>, <Thawing of Cell Sheet>, and <Transplantation after Thawing> were performed in the same manner as in Example 26, except that the set temperature of the defroster of Example 27 was set to 20°C. The thawing time at this time was 5 minutes. After thawing, the appearance of the cell sheet was good.

### [Example 29]

<Production of Cell Sheet>, <Cryopreservation of Cell Sheet>, <Thawing of Cell Sheet>, and <Transplantation after Thawing> were performed in the same manner as in Example 26, except that the set temperature of the defroster of Example 27 was set to 4°C. The thawing time at this time was 8 minutes. After thawing, the appearance of the cell sheet was good.

### [Example 30]

<Production of Cell Sheet>, <Cryopreservation of Cell Sheet>, <Thawing of Cell Sheet>, and <Transplantation after Thawing> were performed in the same manner as in Example 26, except that, in the method shown in <Thawing of Cell Sheet> of Example 12, the thawing at room temperature in the safety cabinet was changed to the thawing in a refrigerator set to 4°C. The thawing time at this time was 37 minutes. After thawing, the appearance of the cell sheet was good.

The appearance of the cell sheet, the measurement of the cell viability, and the transplantation after thawing were evaluated for Examples 27 to 30. The results are shown in Table 5. It is noted that, for reference, the results of Examples 4 and 12 are shown again in Table 5.

### [Table 5]

**Table 5**

| Example | Thawing Time (min) | Evaluation | | |
|---|---|---|---|---|
| | | Appearance of Cell Sheet After Thawing | Fluctuation Ratio of Cell Activity Before/After Freezing (%) | Transplantation After Thawing |
| Example 4 | 16 | Good | 89 | Good |
| Example 12 | 18 | Good | 117 | Good |
| Example 27 | 3 | Good | 102 | Good |
| Example 28 | 5 | Good | 89 | Good |
| Example 29 | 8 | Good | 99 | Good |
| Example 30 | 37 | Good | 63 | Good |

In Examples 4, 12, and 27 to 30, there was no problem with the appearance of the cell sheet. In addition, Examples 4, 12, and 27 to 30 showed a result that the "fluctuation ratio of cell activity before and after freezing" was higher than that in Comparative Example 5. That is, it can be thought that the cell survival rate after freezing can be maintained at a high level. Among these, it was found that Examples 4, 12, and 27 to 29 had a higher "fluctuation ratio of cell activity before and after freezing" than Example 30 in which the freezing time was relatively long. In addition, in any of Examples, the transplantation after thawing could be performed well.

It was confirmed that the same results as in Examples 1 to 30 could be obtained even in a case where mouse fibroblasts were used instead of human fibroblasts.

Priority is claimed based on Japanese Patent Application No. 2023-115623 filed on July 14, 2023, and Japanese Patent Application No. 2023-220506 filed on December 27, 2023, the entire disclosures of which are incorporated herein by reference.

### REFERENCE SIGNS LIST

- 10: culturing carrier substrate
- 12: culturing surface (surface)
- 14: non-culturing surface (back surface)
- 20: culture vessel
- 30: culture liquid
- 40: cell sheet
- 50: culturing carrier substrate with cell sheet
- 60: cryopreservation solution
- 70: transplantation site

## Claims

1. A transplantation method comprising:
a culturing step of culturing a cell sheet on a culturing surface of a culturing carrier substrate; and
a transplantation step of, after attaching the cell sheet formed over the culturing surface of the culturing carrier substrate to a transplantation site, peeling off the culturing carrier substrate from the cell sheet.

2. The transplantation method according to Claim 1,
wherein a substrate thickness of the culturing carrier substrate is equal to or more than 5 µm and equal to or less than 250 µm,
the culturing carrier substrate is a polyether ether ketone film or a polyethylene terephthalate film, and
the culturing surface of the culturing carrier substrate is subjected to a hydrophilization treatment.

3. The transplantation method according to Claim 1 or 2,
wherein a substrate thickness of the culturing carrier substrate is more than 10 µm, and
in the culturing step, the culturing carrier substrate functions as a self-supporting film without being fixed to an inside of a culture vessel.

4. The transplantation method according to any one of Claims 1 to 3,
wherein in the transplantation step, a time from the attachment to the peeling off is equal to or less than 10 minutes.

5. The transplantation method according to any one of Claims 1 to 4,
wherein an area of the cell sheet obtained by the culturing step is equal to or more than 0.3 cm².

6. A culturing carrier substrate used for transporting a cell sheet cultured over a culturing surface,
wherein the culturing carrier substrate has a substrate thickness equal to or more than 5 µm and equal to or less than 250 µm.

7. The culturing carrier substrate according to Claim 6,
wherein the substrate thickness of the culturing carrier substrate is more than 10 µm, and
the culturing carrier substrate functions as a self-supporting film without being fixed to an inside of a culture vessel during culturing of the cell sheet.

8. The culturing carrier substrate according to Claim 6 or 7,
wherein the culturing surface is subjected to a hydrophilization treatment.

9. The culturing carrier substrate according to Claim 8,
wherein the hydrophilization treatment is a plasma treatment.

10. The culturing carrier substrate according to any one of Claims 6 to 9,
wherein the culturing carrier substrate is a polyether ether ketone film or a polyethylene terephthalate film.

11. The culturing carrier substrate according to any one of Claims 6 to 10,
wherein the culturing surface does not contain a temperature-responsive polymer.

12. A package in which the culturing carrier substrate according to any one of Claims 6 to 11 is packaged with a packaging material.

13. A culturing carrier substrate with a cell sheet, comprising:
a laminate of the culturing carrier substrate according to any one of Claims 6 to 11 and a cell sheet,
wherein the cell sheet is in a state of covering at least 50% of a surface of the culturing carrier substrate on a culturing surface side.

14. The culturing carrier substrate with a cell sheet according to Claim 13,
wherein an area of the cell sheet is equal to or more than 0.3 cm².

15. A method of manufacturing a culturing carrier substrate with a cell sheet, the method comprising:
a culturing step of introducing a plurality of cells, a culture medium, and the culturing carrier substrate according to any one of Claims 6 to 11 into an inside of a culture vessel to culture a cell sheet over a culturing surface of the culturing carrier substrate; and
an extraction step of extracting the culturing carrier substrate with the cell sheet from the culture vessel.

16. The method of manufacturing a culturing carrier substrate with a cell sheet according to Claim 15,
wherein in the culturing step, the cell sheet is cultured without fixing the culturing carrier substrate to the inside of the culture vessel.

17. The method of manufacturing a culturing carrier substrate with a cell sheet according to Claim 15 or 16,
wherein in the extraction step, an operation of separating the culturing carrier substrate from the culture vessel by a physical operation is not required.

18. A cryopreservation method comprising a cooling step of cooling, in a cryopreservation solution, a culturing carrier substrate with a cell sheet, said culturing carrier substrate with a cell sheet including a culturing carrier substrate and a cell sheet formed on a surface of the culturing carrier substrate.

19. The cryopreservation method according to Claim 18,
wherein in the cooling step, the culturing carrier substrate with a cell sheet is cooled in the cryopreservation solution using a non-flow-through-type cooling device.

20. The cryopreservation method according to Claim 18 or 19, wherein in the cooling step, a cooling rate at 0°C to -5°C is equal to or more than 0.1°C/min and equal to or less than 15°C/min.

21. The cryopreservation method according to any one of Claims 18 to 20, further comprising:
a cryopreservation step of storing the culturing carrier substrate with a cell sheet at -196°C to -60°C after the cooling step.

22. A frozen product of a culturing carrier substrate with a cell sheet, the frozen product comprising:
a culturing carrier substrate; and
a cell sheet formed on a surface of the culturing carrier substrate,
wherein the culturing carrier substrate and the cell sheet are in a cryopreserved state.

23. The frozen product of a culturing carrier substrate with a cell sheet according to Claim 22,
wherein the frozen product is in a cryopreserved state at -196°C to -60°C.

24. The frozen product of a culturing carrier substrate with a cell sheet according to Claim 22,
wherein the frozen product is in a cryopreserved state at -196°C to -135°C.

25. The frozen product of a culturing carrier substrate with a cell sheet according to Claim 22,
wherein the frozen product is in a cryopreserved state at -134°C to -60°C.

26. A package in which the frozen product according to any one of Claims 22 to 25 is packaged with a packaging material.
